# EUROPEAN PATENT APPLICATION

(11) **EP 4 458 813 A1**
(43) Date of publication of application: **06.11.2024**
(21) Application number: 22915042.0
(22) Date of filing: 29.12.2022
(51) Int. Cl.: C07D 239/545, C07D 239/56, C07D 401/06, C07D 401/14, C07D 239/48, A61K 31/513, A61P 7/02, A61P 9/10, A61P 25/00

(54) **MULTI-SUBSTITUTED URACIL DERIVATIVE AND PREPARATION METHOD THEREFOR AND APPLICATION THEREOF**

(30) Priority: 29.12.2021 CN 202111641890
(71) Applicant: Hangzhou Adamerck Pharmlabs Inc., Hangzhou, Zhejiang 311112 (CN)
(72) Inventor: QI, Youmao, Hangzhou, Zhejiang 311112 (CN)
(74) Representative: Mewburn Ellis LLP
(86) International application number: PCT/CN2022/143340
(87) International publication number: WO 2023/125792

(57) **Abstract**

The present invention relates to a multi-substituted uracil derivative and a preparation method therefor and an application thereof. Specifically, the compound of the present invention has a structure as shown in formula (I) in which the definitions of groups and substituents are as stated in the description. Futher disclosed in the present invention is a preparation method for the compound and a use of the compound in antithrombotic, treatment of cardiovascular and cardiovascular diseases, ect.

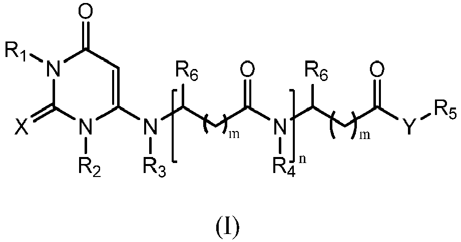

## Description

### Technical field

The present invention relates to the field of pharmaceuticals, and specifically to a multi-substituted uracil derivative and preparation method therefor and application thereof.

### Background

Thrombosis can occur at any age and at any time, with high rates of disability and death, seriously threatening human life and health. According to statistics, globally about 18 million people die of various thrombotic diseases every year, and the deaths caused by thrombotic diseases accounted for 51% of the global total deaths, ranking first in the global total mortality rate. In China, there are 1.5 million new thrombosis patients every year, and 2.5 million people suffer from thrombosis diseases every year, 70% of which will have sequelae.

Currently, commonly used drugs for the prevention and treatment of thrombosis can be used to inhibit or reverse the formation of thrombosis by intervening in different stages of the coagulation process (e.g., inhibiting activity of coagulation factor, inhibiting platelet aggregation, etc.). For a long time, antithrombotic drugs have made great progress in terms of efficacy and other aspects. However, all antithrombotic drugs in the clinic, whether they are old drugs used for decades or new antithrombotic drugs marketed in recent years, have different degrees of adverse reactions, of which the risk of bleeding is a common defect of all types of antithrombotic drugs, and may even become a life-threatening risk of malignancy. This defect limits the clinical application of such drugs and seriously threatens the medication safety of patients. There is no satisfactory clinical countermeasures to this defect. Most drugs (e.g., clopidogrel) currently have no specific antidote, and severe bleeding can only be resuscitated by platelet supplementation and fresh clotting factors when it occurs.. Therefore, there is a growing market demand for mild antithrombotic drugs in the clinic, and they have a wide range of applications in the prevention and treatment of cardiac, cerebral, and other arterial circulatory disorders caused by high platelet aggregation, such as recent stroke, myocardial infarction, and confirmed peripheral arterial disease.

### Summary

The purpose of the present invention is to provide a compound of Formula I and a preparation method therefor and its use in antithrombosis.

In the first aspect of the present invention, provided is a compound, and the compound is a compound of Formula I, or a pharmaceutically acceptable salt, a solvate, a hydrate, an isomer, or a prodrug thereof, 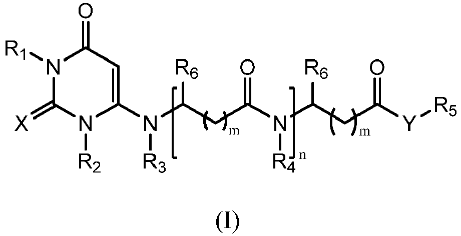 wherein,
R₁ and R₂ are each independently selected from the group consisting of H, substituted or unsubstituted C1-C10 alkyl, C1-C10 haloalkyl, C3-C10 cycloalkyl, C3-C10 halocycloalkyl, C1-C10 alkoxy, C1-C10 haloalkoxy, -(C=O)-(C1-C10 alkyl), C2-C10 alkenyl, C2-C10 alkynyl, halogen, substituted or unsubstituted 5-10 membered heterocycloalkyl containing 1, 2 or 3 heteroatoms selected from N, O and S, substituted or unsubstituted C6-C10 aryl, substituted or unsubstituted 5-10 membered heteroaryl containing 1, 2 or 3 heteroatoms selected from N, O and S, -(C1-C6 alkylene)-(substituted or unsubstituted 5-10 membered heteroaryl containing 1, 2 or 3 heteroatoms selected from N, O and S), and -(C1-C6 alkylene)-(substituted or unsubstituted C6-C10 aryl);
X is selected from the group consisting of: O, S and NH;
Y is O;
R₃ is selected from the group consisting of: H and C1-C10 alkyl;
R₄ is selected from the group consisting of: H and C1-C10 alkyl;
R₅ is selected from the group consisting of: H, C1-C10 alkyl, substituted or unsubstituted C3-C10 cycloalkyl, substituted or unsubstituted C6-C10 aryl, substituted or unsubstituted 5-10 membered heterocycloalkyl containing 1, 2, or 3 heteroatoms selected from N, O, and S, and substituted or unsubstituted C5-C12 bridged cycloalkyl;
each R₆ is independently selected from the group consisting of: H, C1-C10 alkyl, -(C1-C6 alkylene)- (substituted or unsubstituted C6-C10 aryl);
each m is independently selected from the group consisting of: 0, 1, 2, 3, 4, 5 and 6;
n is selected from the group consisting of: 1, 2, 3 and 4;
the "substituted" each independently refers to being substituted by one or more substituents selected from the group consisting of: C1-C10 alkyl, -(C1-C6 alkylene)-piperazinyl, -(C1-C6 alkylene)- 5-10-membered heteroaryl containing 1, 2 or 3 heteroatoms selected from N, O and S, C1-C10 haloalkyl, C1-C10 alkoxy, C1-C10 alkylthiol, CN, nitro, -NRₐR_{b}, -(C=O)-NRₐR_{b}, halogen, hydroxy and oxo;
Rₐ and R_{b} are each independently selected from the group consisting of: H, C1-C10 alkyl, and C1-C10 haloalkyl;
optionally, the alkylene within the bracket is substituted with 1, 2 or 3 OH.

In another preferred embodiment, R₁ and R₂ are each independently selected from the group consisting of: H, substituted or unsubstituted C1-C6 alkyl, C1-C6 haloalkyl, C3-C8 cycloalkyl, C3-C8 halocycloalkyl, C1-C6 alkoxy, C1-C6 haloalkoxy, -(C=O)-(C1-C6 alkyl), C2-C6 alkenyl, C2-C6 alkynyl, halogen, substituted or unsubstituted 5-10 membered heterocycloalkyl containing 1, 2 or 3 heteroatoms selected from N, O and S, substituted or unsubstituted C6-C10 aryl, substituted or unsubstituted 5-10 membered heteroaryl containing 1, 2 or 3 heteroatoms selected from N, O and S, -(C1-C6 alkyene)-(substituted or unsubstituted 5-10 membered heteroaryl containing 1, 2 or 3 heteroatoms selected from N, O and S), and -(C1-C6 alkylene)-(substituted or unsubstituted C6-C10 aryl);
X is selected from the group consisting of: O, S and NH;
Y is O;
R₃ is selected from the group consisting of: H and C1-C6 alkyl;
R₄ is selected from the group consisting of: H and C1-C6 alkyl;
R₅ is selected from the group consisting of: H, C1-C6 alkyl, substituted or unsubstituted C3-C6 cycloalkyl, substituted or unsubstituted C6-C10 aryl, substituted or unsubstituted 5-10 membered heterocycloalkyl containing 1, 2, or 3 heteroatoms selected from N, O and S, and substituted or unsubstituted C5-C12 bridged cycloalkyl;
each R₆ is independently selected from the group consisting of: H, C1-C6 alkyl, and -(C1-C6 alkylene)- (substituted or unsubstituted C6-C10 aryl);
each m is independently selected from the group consisting of: 0, 1, 2, 3, 4, 5 and 6;
n is selected from the group consisting of: 1, 2, 3 and 4;
the "substituted" each independently refers to being substituted by one or more substituents selected from the group consisting of: C1-C6 alkyl, -(C1-C6 alkylene)-piperazinyl, -(C1-C6 alkylene)- 5-10-membered heteroaryl containing 1, 2 or 3 heteroatoms selected from N, O and S, C1-C6 haloalkyl, C1-C6 alkoxy, C1-C6 alkylthiol, CN, nitro, - NRₐR_{b}, -(C=O)-NRₐR_{b}, halogen, hydroxy and oxo;
Rₐ and R_{b} are each independently selected from the group consisting of: H, C1-C6 alkyl, and C1-C6 haloalkyl;
optionally, the alkylene within the bracket is substituted with 1 OH.

In another preferred embodiment, R₁ and R₂ are each independently selected from the group consisting of: H, substituted or unsubstituted C1-C6 alkyl, -(C1-C6 alkylene)-(substituted or unsubstituted 5-10-membered heteroaryl containing 1, 2 or 3 heteroatoms selected from N, O and S), and -(C1-C6 alkylene)-(substituted or unsubstituted C6-C10 aryl);
X is selected from the group consisting of: O, S and NH;
Y is O;
R₃ is selected from the group consisting of: H and C1-C6 alkyl;
R₄ is H;
R₅ is selected from the group consisting of: H, C1-C6 alkyl, substituted or unsubstituted C3-C6 cycloalkyl, substituted or unsubstituted C6-C10 aryl, and substituted or unsubstituted C5-C12 bridged cycloalkyl;
each R₆ is independently selected from the group consisting of: H, C1-C6 alkyl, -(C1-C6 alkylene)- (substituted or unsubstituted C6-C10 aryl);
each m is independently selected from the group consisting of: 0, 1, 2, 3, 4, 5 and 6;
n is selected from the group consisting of: 1, 2, 3 and 4;
the "substituted" each independently refers to being substituted by one or more substituents selected from the group consisting of: C1-C6 alkyl, C1-C6 haloalkyl, CN and halogen.

In another preferred embodiment, R₁ and R₂ are each independently selected from the group consisting of: H, methyl, ethyl, propyl, isopropyl, butyl, isobutyl, tert-butyl, neopentyl, tert-pentyl, -CH₂-(1-to-6 R'-substituted or unsubstituted phenyl) and -CH₂-pyridyl;
each R' is independently selected from the group consisting of: methyl, ethyl, propyl, isopropyl, butyl, isobutyl, tert-butyl, neopentyl, tert-pentyl, F, Cl, Br, I and CN.

In another preferred embodiment, -CH₂-pyridinyl is selected from the group consisting of: pyridinyl with -CH₂-N located at ortho-position, pyridinyl with -CH₂-N located at meta-position, and pyridinyl with -CH₂-N located at para-position.

In another preferred embodiment, X is O or S.

In another preferred embodiment, R₃ is H;
R₄ is H;
R₅ is selected from the group consisting of: H, methyl, ethyl, propyl, isopropyl, butyl, tert-butyl, neopentyl, tert-pentyl, and the following groups: 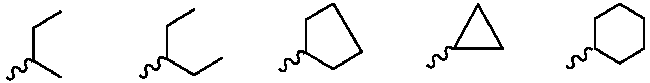 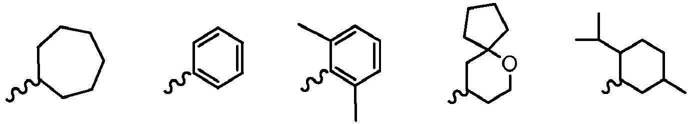 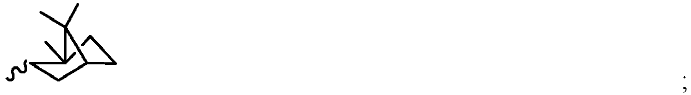
wherein 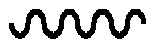 represents a linking bond to Y.

In another preferred embodiment, the compound is selected from the group consisting of:

| No. | R₁ | R₂ | X | R₃ | R₆ | R₄ | Y | R₅ | m | n |
|---|---|---|---|---|---|---|---|---|---|---|
| 1-1 | CH₃ | CH₃ | O | H | H,H | H | O | CH₂CH₃ | 1,1 | 1 |
| 1-2 | CH₃ | CH₃ | O | H | H,H | H | O | CH(CH₃)₂ | 1,1 | 1 |
| 1-3 | CH₃ | CH₃ | O | H | H,H | H | O | H | 1,1 | 1 |
| 1-4 | CH₃ | CH₃ | O | H | H,H | H | O | 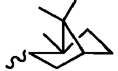 | 1,1 | 1 |
| 1-5 | CH₃ | CH₃ | O | H | H,H | H | O | 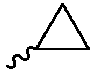 | 1,1 | 1 |
| 1-6 | CH₃ | CH₃ | O | H | H,H | H | O | 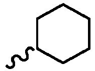 | 1,1 | 1 |
| 1-7 | CH₃ | CH₃ | O | H | H,H | H | O | 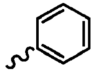 | 1,1 | 1 |
| 1-8 | CH₃ | CH₃ | O | H | H,H | H | O | 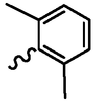 | 1,1 | 1 |
| 1-9 | CH₃ | CH₃ | S | H | H,H | H | O | CH₂CH₃ | 1,1 | 1 |
| 1-10 | CH₃ | CH₃ | S | H | H,H | H | O | H | 1,1 | 1 |
| 1-11 | CH₃ | CH₃ | S | H | H,H | H | O | 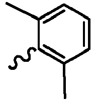 | 1,1 | 1 |
| 1-12 | CH₃ | CH₃ | O | H | H,H | H | O | 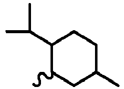 | 1,1 | 1 |
| 1-13 | CH₃ | 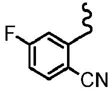 | O | H | H,H | H | O | CH(CH₃)₂ | 1,1 | 1 |
| 1-14 | CH₃ | 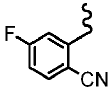 | O | H | H,H | H | O | H | 1,1 | 1 |
| 1-15 | CH₃ | 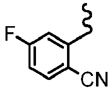 | O | H | H,H | H | O | 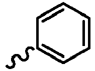 | 1,1 | 1 |
| 1-16 | CH₃ | 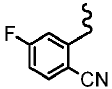 | O | H | H,H | H | O | 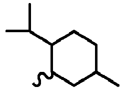 | 1,1 | 1 |
| 1-17 | CH₃ | 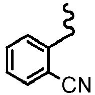 | O | H | H,H | H | O | CH₂CH₃ | 1,1 | 1 |
| 1-18 | CH₃ | 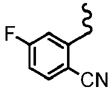 | O | H | H,H | H | O | CH₂CH₃ | 1,1 | 1 |
| 1-19 | CH₃ | 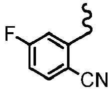 | O | H | H,H | H | O | 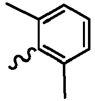 | 1,1 | 1 |
| 1-22 | CH₃ | CH₃ | O | H | 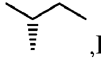 H | H | O | CH₂CH₃ | 0,1 | 1 |
| 1-23 | CH₃ | CH₃ | O | H | H,H | H | O | CH₂CH₃ | 4,4 | 1 |
| 1-24 | CH₃ | CH₃ | O | H | H,H | H | O | H | 4,4 | 1 |
| 1-26 | C₆H₅C H2 | C₆H₅CH₂ | O | H | H,H | H | O | CH(CH₃)₂ | 1,1 | 1 |
| 1-27 | CH₃ | 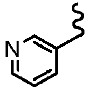 | O | H | H,H | H | O | CH(CH₃)₂ | 1,1 | 1 |
| 1-28 | CH₃ | C₆H₅CH₂ | O | H | H,H | H | O | CH₂CH₃ | 1,1 | 1 |
| 1-29 | CH₃ | C₆H₅CH₂ | O | H | H,H | H | O | H | 1,1 | 1 |
| 1-30 | CH₃ | C₆H₅CH₂ | O | H | H,H | H | O | 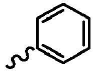 | 1,1 | 1 |
| 1-31 | CH₃ | 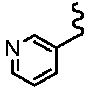 | O | H | H,H | H | O | H | 1,1 | 1 |
| 1-32 | CH₃ | 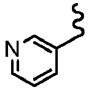 | O | H | H,H | H | O | 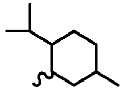 | 1,1 | 1 |
| 1-34 | 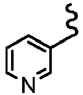 | 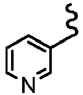 | O | H | H,H | H | O | CH(CH₃)₂ | 1,1 | 1 |
| 1-37 | CH₃ | CH₃ | O | H | 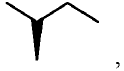 H | H | O | CH₂CH₃ | 0,1 | 1 |
| 1-38 | H | H | O | H | H,H | H | O | CH₂CH₃ | 4,4 | 1 |
| 1-39 | H | H | N H | H | H,H | H | O | CH₂CH₃ | 1,1 | 1 |
| 1-42 | CH₃ | CH₃ | O | H | 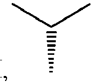 H | H | O | CH₂CH₃ | 1,0 | 1 |
| 1-45 | CH₃ | 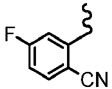 | O | CH₃ | H,H | H | O | CH(CH₃)₂ | 1,1 | 1 |
| 1-48 | CH₃ | CH₃ | O | H | H,H | H | O | CH₂CH₃ | 3,3 | 2 |
| 1-49 | CH₃ | CH₃ | O | H | 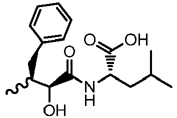 | | | | | |
| 1-54 | 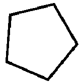 | 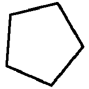 | O | H | H,H | H | O | CH₂CH₃ | 1,1 | 1 |
| 1-55 | 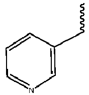 | 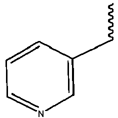 | O | H | H,H | H | O | CH₂CH₃ | 1,1 | 1 |

In another preferred embodiment, in the above table, R₆ and m are both groups defined from left to right in Formula I.

In the second aspect of the present invention, provided is a method for preparing the compound described in the first aspect of the present invention, comprising steps of: 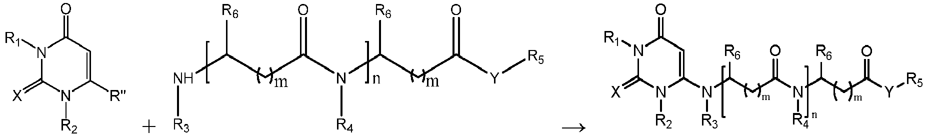 reacting 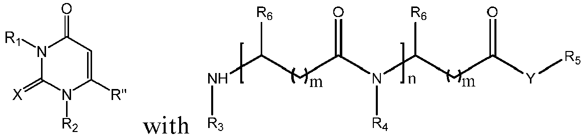 to obtain the compound of formula I;
wherein,
R" is halogen;
R₁, R₂, R₃, R₄, R₅, R₆, X, Y, m and n are as defined in the first aspect of the present invention.

In the third aspect of the present invention, provided is a pharmaceutical composition, comprising pharmaceutically acceptable carriers and a safe and effective amount of one or more compounds described in the first aspect of the present invention.

In another preferred embodiment, the dosage form of the pharmaceutical composition is selected from the group consisting of: tablets, capsules, granules, oral liquids, lyophilized powders and injections.

In another preferred embodiment, the dosage form is sustained release and/or controlled release and/or enteric soluble.

In the fourth aspect of the present invention, provided is a use of the compound described in the first aspect of the present invention, which is selected from the group consisting of:
1) in the preparation of a medication for the treatment of infarction-related diseases;
2) in the preparation of a medication for thromboprophylaxis and/or thrombolytic therapy for thrombotic diseases, anti-edema therapy and/or immuno-anti-inflammatory therapy ;
3) in the preparation of a medication for the improvement of neuronal damage resulting from traumatic brain injury, cerebral hemorrhage, and/or brain tumor surgery.

In another preferred embodiment, the infarction-related diseases are selected from the group consisting of: neuronal damage of neural tissue resulting from acute ischemic stroke (cerebral infarction), myocardial infarction, and pulmonary embolism; and/or
the thrombotic diseases are selected from the group consisting of: cardiovascular diseases, cerebrovascular diseases, and peripheral vascular diseases.

It should be understood that within the scope of the present invention, the above-mentioned technical features of the present invention and the technical features specifically described in the following (such as embodiments) can be combined with each other to form a new or preferred technical solution. Limited to space, It will not be repeated here.

### Detailed Description of the Invention

After long-term and in-depth research, the present inventor has unexpectedly prepared a compound with excellent anti-thrombotic effect and high safety. On this basis, the present invention is completed.

### Terms

In the present invention, unless otherwise specified, the terms used herein have the ordinary meanings known to those skilled in the art.

In the present invention, the term "halogen" refers to F, Cl, Br or I.

In the present invention, "C1-C6 alkyl" refers to a straight or branched alkyl comprising 1-to-6 carbon atoms, such as methyl, ethyl, propyl, isopropyl, butyl, isobutyl, tert-butyl, neopentyl, tert-pentyl, and the like. The term "C1-C10 alkyl" has a similar meaning.

In the present invention, the term "C2-C6 alkenyl" refers to a straight or branched chain alkenyl containing a double bond and 2-to-6 carbon atoms, and non-limitingly includes vinyl, propenyl, butenyl, isobutenyl, pentenyl, hexenyl, and the like.

In the present invention, the term "C2-C6 alkynyl" refers to a straight or branched alkynyl containing a triple bond and 2-to-6 carbon atoms, and non-limitingly includes ethynyl, propynyl, butynyl, isobutynyl, pentynyl, hexynyl and the like.

In the present invention, the term "C3-C8 cycloalkyl" refers to a cyclic alkyl having 3-8 carbon atoms on the ring, and non-limitingly includes cyclopropyl, cyclobutyl, cyclopentyl, cyclohexyl, cycloheptyl, cyclooctyl, and the like. The term "C3-C10 cycloalkyl" has a similar meaning.

In the present invention, the term "C1-C6 alkoxy" refers to straight or branched alkoxy having 1-to-6 carbon atoms, and non-limitingly includes methoxy, ethoxy, propoxy, isopropoxy, butoxy and the like. Preferably, C1-C4 alkoxy. The term "C1-C10 alkoxy" has a similar meaning.

The term "C1-C10 alkylthiol" is -S-(C1-C10 alkyl). In the present invention, the term "heterocyclyl" refers to a 4-8-membered heterocyclyl containing 1, 2, or 3 heteroatoms selected from N, O and S, including, but not limited to the following groups:

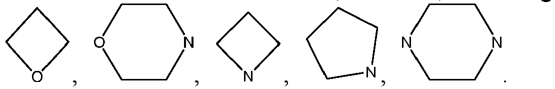

In the present invention, the terms "aryl ring" or "aryl" have the same meaning, preferably "C6-C10 aryl". The term "C6-C10 aryl" refers to an aromatic ring group having 6 to 10 carbon atoms on the ring without heteroatoms, such as phenyl, naphthyl and the like.

In the present invention, the terms "aromatic heterocycle" or "heteroaryl" have the same meaning and refer to a heteroaromatic group containing one to more heteroatoms. For example, "C3-C10 heteroaryl" refers to an aromatic heterocyclic ring containing 1 to 4 heteroatoms selected from oxygen, sulfur, and nitrogen and 3 to 10 carbon atoms. Non-limiting examples include: furanyl, thienyl, pyridinyl, pyrazolyl, pyrrolyl, N-alkylpyrrolyl, pyrimidinyl, pyrazinyl, imidazolyl, tetrazolyl and the like. Said heteroaryl ring may be condensed to an aryl, a heterocyclyl or a cycloalkyl ring, wherein the ring attached to the parent structure is a heteroaryl ring. The heteroaryl group may be optionally substituted or unsubstituted.

In the present invention, the term "halogenated(halo)" means being substituted by deuterium.

In the present invention, the term "deuterated" means being substituted by deuterium.

In the present invention, the term "substituted" refers to that one or more hydrogen atoms on a particular group are substituted by a particular substituent. The particular substituent is a substituent described above correspondingly, or a substituent appeared in the Examples. Unless specifically stated, a particular substituted group may have a substituent selected from a particular group at any substitutable site of the group, and the substituents may be the same or different at various positions. It should be understood by those skilled in the art that the combination of substituents contemplated by the present invention are those that are stable or chemically realizable. the substituents are, for example (but not limited to): halogen, hydroxyl, carboxy (-COOH), C1-C6 alkyl, C2-C6 alkenyl, C2-C6 alkynyl, C3-C8 cycloalkyl, 3- to 12-membered heterocyclyl, aryl, heteroaryl, C1-C8 aldehydes, C2-C10 acyl, C2-C10 ester, amino, C1-C6 alkoxy, C1-C10 sulfonyl, etc.

In the present invention, the term "1-6" refers to 1, 2, 3, 4, 5 or 6. Other similar terms independently have similar meanings. The term "more" means 2-to-6, such as 2, 3, 4, 5 or 6.

The term "carbonyl" has a structure of 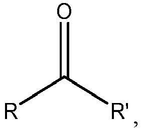 and R and R' may independently represent hydrogen, alkyl or substituted alkyl, cycloalkyl or substituted cycloalkyl, aryl or substituted aryl, heterocycle or substituted heterocycle, as defined above.

It should be understood that when a group is present in several different positions of a compound, its definition in each position is independent of each other and may be the same or different. That is, the term "selected from the group consisting of:" has the same meaning as the term "each independently selected from the group consisting of:".

### Compound and preparation method therefor

The present invention provides a compound of formula I, 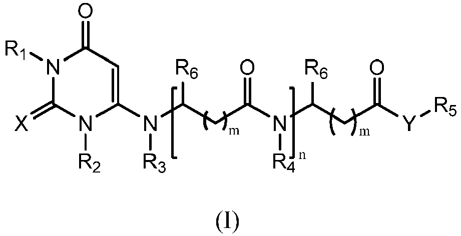 wherein, each group is as defined above.

In another preferred embodiment, any one of R₁, R₂, R₃, R₄, R₅, R₆, X, Y, m and n is independently the corresponding group in the specific compound described in the present invention.

As used herein, the term "pharmaceutically acceptable salt" refers to a salt suitable for use as a drug and formed by the compound of the invention and an acid or a base. Pharmaceutically acceptable salts include inorganic and organic salts. A preferred class of salts are those formed by the compound of the present invention with acids. Acids suitable for forming salts include, but are not limited to: inorganic acids such as hydrochloric, hydrobromic, hydrofluoric, sulphuric, nitric, and phosphoric acids, etc.; organic acids such as formic, acetic, trifluoroacetic, propionic, oxalic, malonic, succinic, fumaric, maleic, lactic, malic, tartaric, citric, picric, benzoic, methanesulphonic, ethanesulfonic, p-toluenesulphonic, benzenesulphonic, naphthalenesulphonic acids, etc.; and amino acids such as proline, phenylalanine, aspartic acid, glutamic acid, etc..

Another preferred class of salts are salts formed by the compounds of the present invention and a base, such as alkali metal salts (e.g., sodium or potassium salts), alkaline earth metal salts (e.g., magnesium or calcium salts), ammonium salts (e.g., lower alkanolammonium salts, and other pharmaceutically acceptable amine salts), such as methylamine salts, ethylamine salts, propylamine salts, dimethylamine salts, trimethylamine salts, diethylamine salts, triethylamine salts, tert-butylamine salts, ethylenediamine salts, hydroxyethylamine salts, dihydroxyethylamine salts, trihydroxyethylamine salts, and amine salts formed by morpholine, piperazine, and lysine, respectively.

The term "solvate" refers to a complex formed by the coordination of a compound of the present invention with solvent molecules in a specific ratio. "Hydrate" refers to a complex formed by the coordination of a compound of the present invention with water.

In addition, the compounds of the present invention also include prodrugs of the compound of Formula I. The term "prodrug" includes a compound which may be biologically active or inactive by itself and, when taken by suitable means, undergoes metabolism or chemical reactions in the human body and is transformed into a class of compounds of formula I, or a salt or solution consisting of one compound of formula I. The prodrug includes, but is not limited to, forms such as carboxylate, carbonate, phosphate, nitrate, sulphate, sulfone ester, sulfoxide ester, amine compound, carbamate, azo compound, phosphoramide, glucoside, ether, acetal of the compound, and the like.

Exemplarily, the compound has a structure of formula (I): 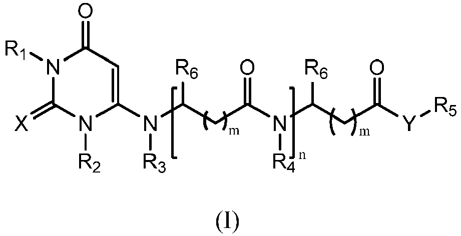 wherein R₁, and R₂ represent any one of hydrogen, various structures of chained and cyclic alkylcontaining short carbon chains with 1-10 carbon atoms, alkyl ketone, alkenyl, alkynyl, fluorine, chlorine, bromine atoms, various heterocycles, benzene rings, phenyl or benzyl mono- or multi-substituted with small molecule groups at various positions of the benzene ring (small molecule groups for substitution include hydrogen, methyl, methylpiperazinyl, ethyl, isopropyl, trifluoromethyl, methoxy, nitrile, methylthiol, nitro, dimethylamino, methylamino, amino, fluorine, chlorine or bromine), or ortho-, meta- or para-ethyl pyridyl, glucosyl, 1,2-propanediol, ((S)-(2,2-dimethyl-1,3-dioxolan-4-yl)methyl, ((R)-(2,2-dimethyl-1,3-dioxolan-4-yl)methylamino)methyl;

Specifically, when R₁ is one of 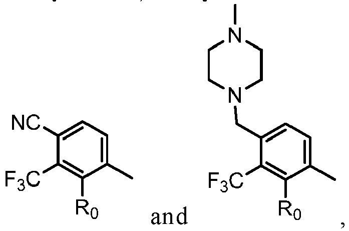 R₂ may also be one of 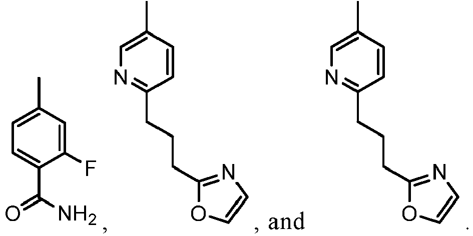 Wherein R₀ represents H, CH₃ or F.
R₃, R₄=H, CH₃, Et;
Rs= H, CH₃, Et, Pr, i-Pr, Bu, I-Bu, and the following groups: 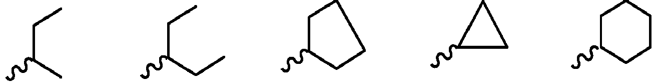 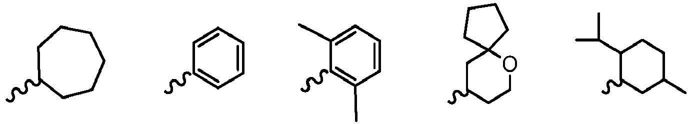 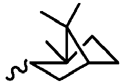
R₆ represents any one of H, L- or D-configuration of chained alkyl of various structures containing short carbon chains with 1-6 carbon atoms, hydroxyalkyl, benzyl, p-hydroxybenzyl, and carboxylate.
X = O, S, NH;
Y = O, NH, NR₀;
R₀ = H, CH₃, Et;
m = one of 0,1,2,3 and 4;
n=one of 1,2,3 and 4.

A new multi-substituted uracil derivative, further comprising the following specific structure: 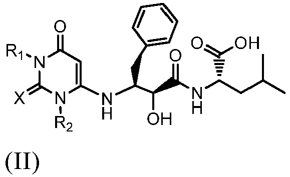 wherein, each group is as defined above.

Typically, in the present invention, formula (I) can be achieved by the following synthesis method:
Bis(p-nitrophenyl) derivative (1) reacts with primary amine derivative to give urea derivative (2), and compound (2) reacts with triphosgene to give diimine derivative (3), which then reacts with malonyl to give barbituric acid derivative (4), or compound (2) reacts directly with dichloromalonyl to give barbituric acid derivative (4). Compound (4) undergoes a halogenation reaction to give 6-halogenated uracil derivative (5), and compound (5) reacts with a series of multi-substituted amino acid ester derivatives (5-A) in the presence of solvent and base to give multi-substituted uracil derivative (I), which further undergoes a hydrolysis reaction to give compound (6). Multi-substituted uracil derivative (I) can also be obtained from the reaction of compound (6) with a phenol or an alcohol.

The reaction formula for the synthesis of (I) is shown below:

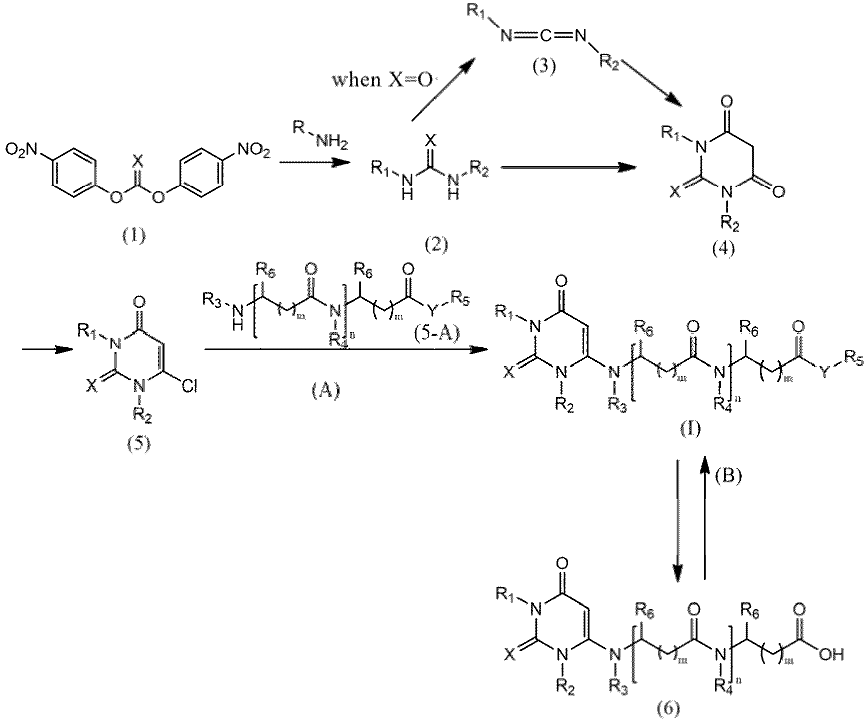

R₁, R₂, R₃, R₄, R₅, R₆, m, n, X and Y are as described above.
wherein, when R₁, and R₂ are the same group, R=R₁=R₂, R-NH₂ may include any one of the following structural formulas, but is not limited to the following structures:

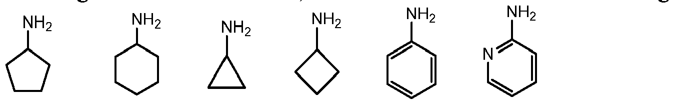

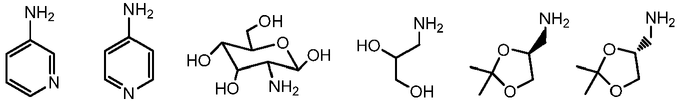

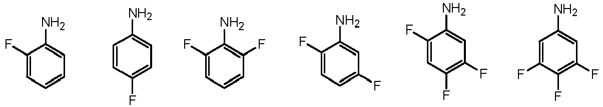

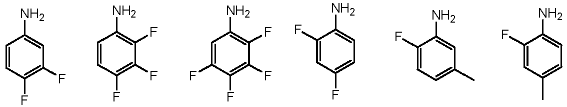

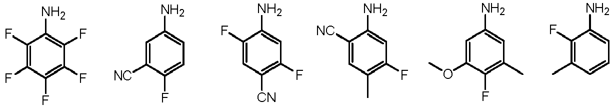

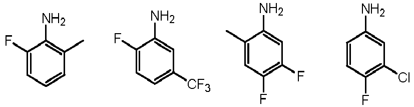

The synthesis of 6-halogenated uracil derivative (5) can also be achieved by the following route: 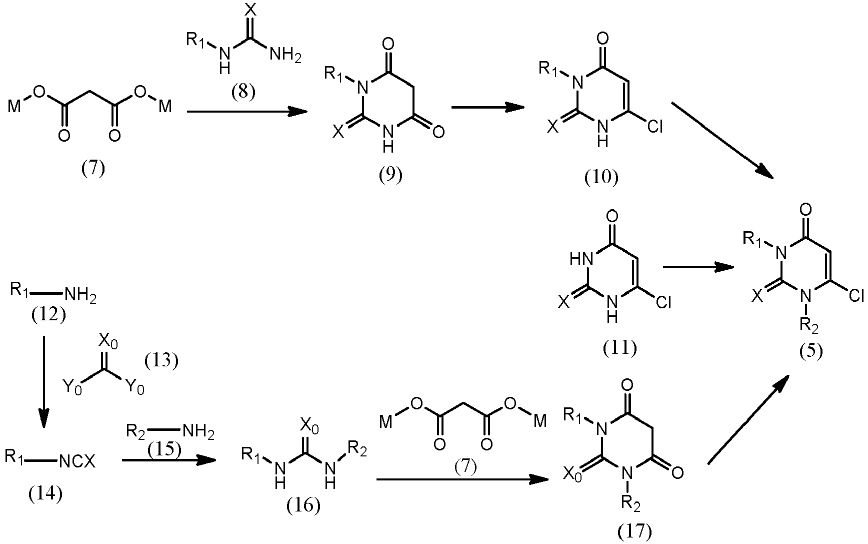 wherein X₀ = one of O and S; Y₀ = one of Cl, Br and OCCl₃, R₁, R₂, M, X and Y are as described above.
(1) when R₁ and R₂ are the same group, Compound (5) can be synthesized directly from compound (11) with halogenated methyl aromatic heterocyclic derivative in the presence of solvent and base.
(2) when R₁ and R₂ are different groups, compound (9) can be obtained by reacting malonate derivative (7) with monosubstituted urea derivative (8), which then undergoes a chlorination reaction to give compound (10), and compound (10) finally undergoes a substitution reaction to give compound (5).
(3) when R₁, R₂ are different groups, compound (14) can be obtained by reacting primary amine analog (12) with phosgene analog (13), which in turn reacts with primary amine analog (15) to obtain urea derivative (16); urea derivative (16) in turn reacts with malonate derivative (7) to obtain barbituric acid derivative (17), which then undergoes a chlorination reaction to give compound (5).

The synthesis of multi-substituted amino acid ester derivative (5-A) can be achieved by the following route:

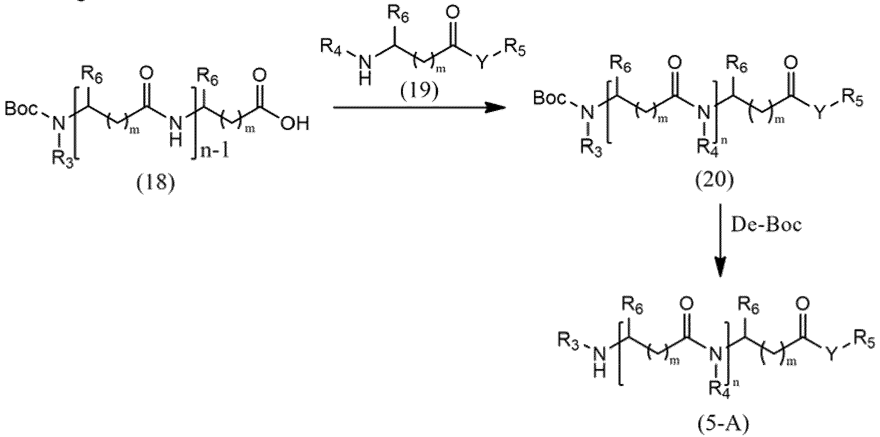

R₄, R₅, R₆, m and Y are as described above.

Compound (20) can be obtained from the reaction of N-Boc-L or D-configuration amino acid or N-Boc alanine derivative (18) with alanine ester derivative or L or D-configuration amino acid ester derivative compounds (19), which then undergoes Boc-deprotection reaction(de-Boc) to obtain multi-substituted amino acid ester derivative (5-A).

Typically, the molar ratio of the 6-halogenated uracil derivative (5) and a series of alanine ester derivatives of different structures in the step is from 0.9 to 1.1: 1, preferably 1:1; the molar ratio of base to 6-halogenated pyrimidine derivative (5) is from 2 to 4:1, preferably 3:1.

Typically, the reaction molar ratio of the compound (11) and the halogenated methyl aromatic heterocyclic derivative in the step is 1:1-1.5.

Typically, the solvent in the step is selected from one of isopropanol, tert-butanol, DMF and DMSO.

Typically, the base in the step is selected from one of diisopropylethylamine, triethylamine, diethylamine, 4-dimethylaminopyridine, potassium carbonate and sodium carbonate.

Typically, L- or D-configuration amino acids in the step include, but are not limited to: one of L-alanine, D-alanine, L-serine, D-serine, L-threonine, D-threonine, L-aspartic acid, D-aspartic acid, L-glutamic acid, D-glutamic acid, L-histidine, D-histidine, L-valine, D-valine, L-leucine, D-leucine, L-isoleucine, D-isoleucine, L-allo-isoleucine, D-allo-isoleucine, L-tryptophan, D-tryptophan, S-methyl-L-cysteine, S-methyl-D-cysteine, L-tyrosine, D-tyrosine, L-phenylalanine and D-phenylalanine.

Typically, the derivative can be used to (a) treat infarction-related diseases, in particular neuronal damage of neural tissue resulting from acute ischemic stroke (cerebral infarction), myocardial infarction, pulmonary embolism, and other related diseases; and/or (b) conduct thromboprophylaxis and thrombolytic therapy, anti-oedema therapy, and immuno-anti-inflammatory therapy for thrombotic disorders; and/or (c) improve neuronal damage resulting from traumatic brain injury, cerebral hemorrhage,or brain tumor surgery.

Typically, the derivative can be used after a cardiovascular infarction and thrombolytic treatment, to achieve an antithrombotic effect and prevent recurrence of the infarction, while reduce the risk of bleeding.

Typically, the compounds may be prepared into slow-release, controlled-release, enteric-coated tablets or capsules, granules, oral liquids, lyophilized powders, injections, etc., by adding pharmaceutically acceptable conventional excipients.

It should be understood that the compound of the present invention can also be conveniently made by optionally combining various synthetic methods described in this specification or known in the art, and such combinations can be readily carried out by those skilled in the field to which the present invention belongs.

It should be understood that the raw materials and reagents used are commercially available unless otherwise specified.

The present invention provides a multi-substituted uracil derivative of formula I and preparation method therefor and application thereof. The compound of formula I can be used to (a) treat infarction-related diseases, in particular neuronal damage of neural tissue resulting from acute ischemic stroke (cerebral infarction), myocardial infarction, pulmonary embolism, and other related diseases; and/or (b) conduct thromboprophylaxis and thrombolytic therapy for thrombotic diseases, anti-oedema therapy, and immuno-anti-inflammatory therapy; and/or (c) improve neuronal damage resulting from traumatic brain injury, cerebral hemorrhage,or brain tumor surgery. In addition, the multi-substituted uracil derivative (I) can be used after a cardiovascular infarction and thrombolytic treatment, to achieve an antithrombotic effect and prevent recurrence of the infarction while reduce the risk of bleeding, which is of great clinical importance. The synthesis method of the multi-substituted uracil derivative of the present invention such as the formula (I) is easy to operate, the conditions are mild, the raw materials are easy to obtain, the yield is high, and the product purity is high.

A series of target pharmaceutical compounds synthesized according to the above reaction are new compounds and their structures are characterized by 1H NMR and ESI-MS.

The results of the animal modeling studies have strongly demonstrated that the multi-substituted uracil derivatives have certain antiplatelet aggregation activity, which is comparable to or superior to that of the corresponding positive control drug, clopidogrel. It was also surprisingly found that the compounds were able to greatly reduce, or even overcome, the hemorrhagic risk of clopidogrel in mouse model experiments, and therefore have the potential to be developed into clinical drugs capable of controlling the hemorrhagic risk during antithrombotic processes.

### Pharmaceutical composition and mode of application

The present invention further provides a pharmaceutical composition comprising pharmaceutically acceptable carriers and a safe and effective amount of one or more compounds of the present invention.

Since the compound of the present invention has excellent antithrombotic activity, the compound of the present invention and its various crystal forms, pharmaceutically acceptable inorganic or organic salts, hydrates or solvates, and pharmaceutical composition containing the compound of the present invention as the main active ingredient can be used for treating, preventing and alleviating embolism related diseases.

The pharmaceutical compositions of the present invention comprise a compound of the present invention or a pharmaceutically acceptable salt thereof within a safe and effective amount range, and pharmaceutically acceptable excipients or carriers. The "safe and effective amount" refers to the amount of compound that is sufficient to significantly improve the condition without causing serious side effects. Typically, the pharmaceutical composition contains 1-2000mg compound of the present invention/dose, and more preferably, 10-1000mg compound of the present invention/dose. Preferably, the "one dose" is a capsule or a tablet.

"Pharmaceutically acceptable carrier" refers to one or more compatible solid or liquid fillers or gel substances, which are suitable for human use and must have sufficient purity and low toxicity. "Compatibility" herein refers to the ability of components of a composition to blend with each other and with the compounds of the invention without significantly reducing the efficacy of the compounds. Examples of pharmaceutically acceptable carriers include cellulose and its derivatives (such as sodium carboxymethyl cellulose, sodium ethyl cellulose, cellulose acetate, etc.), gelatin, talc, solid lubricants (such as stearic acid, Magnesium stearate), calcium sulfate, vegetable oil (such as soybean oil, sesame oil, peanut oil, olive oil, etc.), polyols (such as propylene glycol, glycerin, mannitol, sorbitol, etc.), emulsifier (such as tween^{®}), wetting agents (such as sodium dodecyl sulfate), colorants, flavoring agents, stabilizers, antioxidants, preservatives, non-pyrogen water, etc.

The pharmaceutical composition is in the form of injections, wafers, tablets, pills, powders or granules.

The mode of administration of the compounds or pharmaceutical compositions of the present invention are not particularly limited, and representative mode of administration includes, but is not limited to, oral, intratumoral, rectal, parenteral (intravenous, intramuscular or subcutaneous), and topical administration.

Solid dosage forms for oral administration include capsules, tablets, pills, powders and granules. In these solid dosage forms, the active compound is mixed with at least one conventional inert excipient (or carrier), such as sodium citrate or dicalcium phosphate, or with the following ingredients: (a) filler or compatibilizer, such as starch, lactose, sucrose, glucose, Mannitol and silicic acid; (b) adhesives, such as hydroxymethyl cellulose, alginate, gelatin, polyvinylpyrrolidone, sucrose and gum arabic; (c) moisturizing agents, such as glycerol; (d) disintegrating agents, such as agar, calcium carbonate, potato starch or cassava starch, algic acid, some complex silicates, and sodium carbonate; (e) slow solvent, such as paraffin; (f) absorption accelerators, such as quaternary amine compounds; (g) wetting agents, such as cetyl alcohol and glycerol monostearate; (h) adsorbents, such as kaolin; and (i) lubricants, such as talc, calcium stearate, magnesium stearate, solid polyethylene glycol, sodium dodecyl sulfate, or mixtures thereof. The dosage forms of capsules, tablets and pills may also contain buffers.

Solid dosage forms such as tablets, sugar pills, capsules, pills and granules may be prepared using coating and shell materials such as casings and other materials well known in the art. They may comprise an opacifying agent, and the active compound in such composition or the compound may be released in a delayed manner in a part of the digestive tract. Examples of embedding components that can be employed are polymeric substances and wax substances. If necessary, the active compound may also form a microcapsule form with one or more of the excipients described above.

Liquid dosage forms for oral administration include pharmaceutically acceptable emulsions, solutions, suspensions, syrups and tinctures. In addition to the active compounds, the liquid dosage form may contain inert diluents conventionally used in the art, such as water or other solvents, solubilizers and emulsifiers, for example, ethanol, isopropanol, ethyl carbonate, ethyl acetate, propylene glycol, 1,3-butanediol, dimethylformamide and oils, especially cottonseed oil, peanut oil, corn germ oil, olive oil, castor oil and sesame oil, or mixtures thereof.

In addition to these inert diluents, the composition may also contain auxiliaries such as wetting agents, emulsifiers, suspending agents, sweeteners, flavoring agents and flavors.

In addition to the active compound, the suspension may comprise suspending agents, such as ethoxylated isooctadecanol, polyoxyethylene sorbitol and dehydrated sorbitol esters, microcrystalline cellulose, methanolic aluminum, agar, and any mixtures thereof.

The composition for parenteral injection may comprise physiologically acceptable sterile aqueous or anhydrous solutions, dispersions, suspensions or emulsions, and sterile powders for redissolution into sterile injectable solutions or dispersions. Suitable aqueous and non-aqueous carriers, diluents, solvents, or excipients include water, ethanol, polyols, and suitable mixtures thereof.

Dosage forms of the compound of the invention for topical administration include ointments, powders, patches, propellants and inhalants. The active ingredient is mixed under sterile conditions with physiologically acceptable carriers and any preservatives, buffers or propellants as may be required.

The compound of the present invention can be administered alone or in combination with other pharmaceutically acceptable compounds (such as antithrombotic agents).

The treatment method of the present invention can be administered alone or in combination with other treatment methods or drugs.

When using the pharmaceutical composition, a safe and effective amount of the compound of the present invention is administered to mammals in need of treatment (such as humans), where the dosage at the time of administration is the pharmaceutically considered effective dose, which is typically 1- 2000 mg per day, more preferably 50- 1000mg per day for a 60 kg body weight human. Of course, the specific dosage should also consider the route of administration, the patient's health and other factors, which are within the skill range of skilled doctors.

Compared with the prior art, the main advantages of the present invention include:
(1) The compounds can significantly prolong the coagulation time and shorten the bleeding time in mice injected with ellagic acid via the tail vein;
(2) The compounds can improve cerebral nerve function, reduce the rate of cerebral infarction and cerebral water content in rat cerebral ischemia-reperfusion injury or permanent cerebral ischemia injury, and improve the neuromotor function in rats;
(3) The compounds can improve the degree of cardiomyopathy as well as inhibit the rate of myocardial infarction;
(4) The compounds can reduce the rate of cerebral infarction, cerebral water content and pathological damage in rats with ischemic brain injury caused by autologous thrombus and thrombin, improve the neuromotor function in rats, and exhibit better anti-ischemic brain injury effect, and the efficacy of the compounds is more obvious when used in combination with urokinase;
(5) The compounds have immuno-anti-inflammatory effects;
(6) The compounds have excellent safety and efficacy.

The present invention is further described below in conjunction with specific embodiments. It is to be understood that these examples are intended to illustrate the invention only and not to limit the scope of the invention. The experimental methods that do not indicate specific conditions in the following examples usually follow the conventional conditions, such as those described in Sambrook et al., Molecular Cloning: A Laboratory Manual (New York: Cold Spring Harbor Laboratory Press, 1989), or the conditions suggested by the manufacturer. Unless otherwise specified, percentages and parts are calculated by weight.

Unless otherwise defined, all professional and scientific terms used herein have the same meanings as commonly understood by those skilled in the art. In addition, any methods and materials similar or equivalent to those described herein can be applied to the method of the present invention. The preferred embodiments and materials described herein are for exemplary purposes only.

### Example 1 Preparation of Compound 1-1

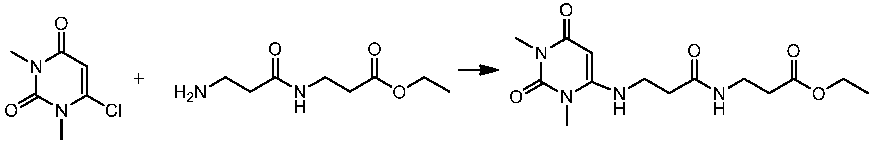

Under the protection of argon, to a reaction flask was added 8.39g of β-alanine-β-alanine ethyl ester (CAS:754933-31-8)(47.76mmol, 1.0eq), 7.0g of 1,3-dimethyl-6-chlorouracil (47.77mmo,1.0eq), 18.52g of N,N-diisopropylethylamine (143.29mmol, 3.0eq) and 42ml of isopropanol, then warmed to 85 °C and stirred until the reaction was complete. Then the reaction stopped heating, and filtrated; the filter cake was pulped with ethanol 100ml at room temperature, and leached; the filter cake was dried in a vacuum at 60 °C to a constant weight to obtain a compound 1-1 as a white powder, with a yield of 78% and a purity of 99.98%.

1H NMR (600 MHz, DMSO-d6)δ1.29(CH3,t,3H), 2.65(CH2,m,2H), 2.66(CH2,m,2H), 3.01(CH3,s,3H), 3.13(CH3,m,3H), 3.16(CH3,q,3H), 3.79(CH2,t,2H), 4.13(CH2,q,2H), 4.50(CH,s,H) ppm. MS:m/z:327.16 (M+1).

### Example 2 Preparation of Compound 1-2

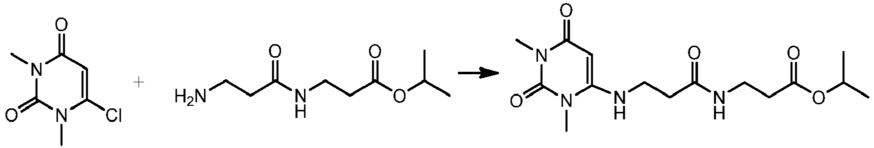

By By referring to the synthesis method of Compound 1-1 in Example 1, 1,3-dimethyl-6-chlorouracil, and compound (CAS:1582394-73-7), were used as raw materials to obtain Compound 1-2 as a beige powder, with a yield of 56.30% and a purity of 97.92%.

1H NMR (600 MHz, DMSO-d6)δ1.32(2CH3,d,6H), 2.65(CH2,m,2H), 2.66(CH2,m,2H), 3.01(CH3,s,3H), 3.13(CH2,m,2H), 3.16(CH3,m,3H), 3.79(CH2,t,2H), 4.50(CH,s,H), 4.93(CH,s,H) ppm. MS:m/z:341.17 (M+1).

### Example 3 Preparation of Compound 1-3

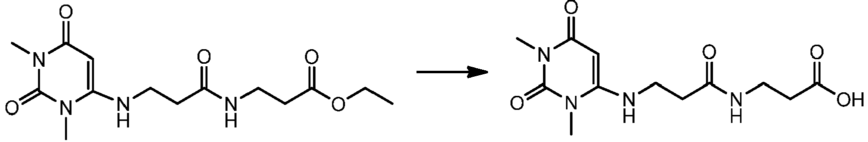

To a 100 mL single-necked flask was added Compound 1-1 (3.8 mmol), 0.23 g of NaOH, 30 mL of ethanol and 3 mL of water. After addition, the reaction was stirred at room temperature. After 2h of stirring, the reaction was sampled and monitored by TLC, which showed that the raw materials in the reaction solution were reacted completely after 2h of stirring. The reaction solution was then adjusted to pH to neutral with concentrated hydrochloric acid, concentrated to dryness directly, and then concentrated with ethanol for several times to obtain compound 1-3 as an off-white powder, with a yield of 90% and a purity of 99.16%.

1H NMR (600MHz, DMSO-d6)δ2.49(CH2,t,2H), 2.66(CH2,t,2H), 3.01(CH3,s,3H), 3.13(CH2,m,2H), 3.16(CH3,m,3H), 3.67(CH2,t,2H), 4.50(CH,s,H)ppm. MS:m/z:299.13 (M+1).

### Example 4 Preparation of Compound 1-4

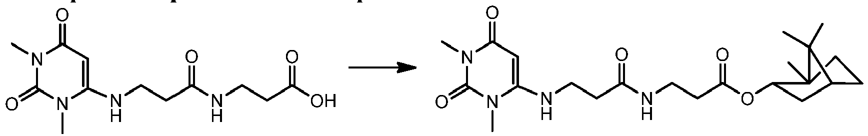

To a 100mL three-necked flask was added 2g of compound 1-3, 20mL of DCM, 1.93g of EDCI, 1.03g of borneol and 0.08g of DMAP, and then stirred at room temperature overnight. The raw materials were still left in the reaction solution, and the reaction continued under stirring until the reaction was completed, and was then diluted with water, extracted; the organic phase was washed with water, then dried with anhydrous sodium sulfate, and then concentrated to dryness to obtain compound 1-4 as a white powder, with a yield of 44.36% and a purity of 98.88%.

1H NMR (600MHz, DMSO-d6)δ0.99(2CH3,s,6H), 1.04(CH3,s,3H), 1.11,1.33(CH2,m,2H), 1.14,1.67(CH2,m,2H), 1.38,1.91(CH2,m,2H), 1.87(CH,m,H), 2.51(CH2,t,2H), 2.66(CH2,t,2H), 3.01(CH3,s,3H), 3.13(CH2,m,2H), 3.16(CH3,m,3H), 3.79(CH2,t,2H), 4.50(CH,s,H), 7.13(CH,t,H), ppm. MS:m/z:435.25(M+1).

### Example 5 Preparation of Compound 1-5

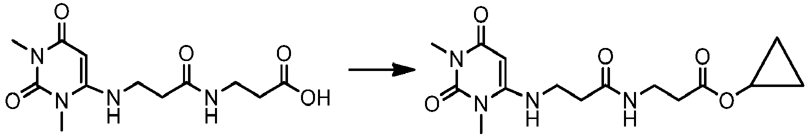

By By referring to the synthesis method of compound 1-4 in Example 4, 4 g of compound 1-3 and 0.93 g of cyclopropanol were used as raw materials to obtain compound 1-5 as a yellow powder, with a yield of 42.91% and a purity of 98.43%.

1H NMR (600 MHz, DMSO-d6)δ0.58;0.34(2CH2,d,4H), 2.65(CH2,m,2H), 2.66(CH2,m,2H), 2.69(CH,m,H), 3.01(CH3,s,3H), 3.13(CH2,m,2H), 3.16(CH3,m,3H), 3.79(CH2,t,2H), 4.50(CH,s,H)ppm. MS:m/z:339.16 (M+1).

### Example 6 Preparation of Compound 1-6

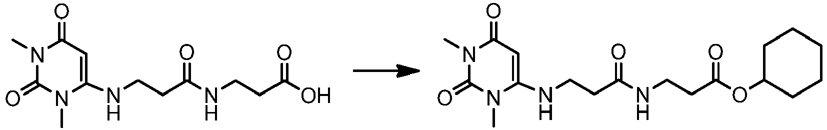

By By referring to the synthesis method of compound 1-4 in Example 4, 4 g of compound 1-3 and 2.01 g of cyclohexanol were used as raw materials to obtain compound 1-6 as a white powder, with a yield of 49.40% and a purity of 99.61%.

1H NMR (600MHz, DMSO-d6)δ1.53;1.43(2CH2,m,4H), 1.49;1.47(CH2,m,2H), 1.80;1.55(2CH2,m,4H), 2.65(CH2,m,2H), 2.66(CH2,m,2H), 3.01(CH3,s,3H), 3.13(CH2,m,2H), 3.16(CH3,m,3H), 3.79(CH2,t,2H), 3.91(CH,m,H), 4.50(CH,s,H)ppm. MS:m/z:381.21 (M+1).

### Example 7 Preparation of Compound 1-7

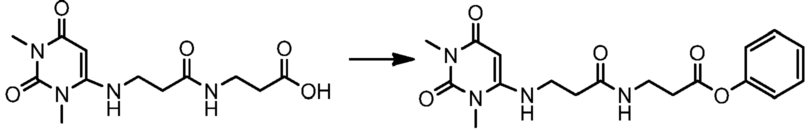

By referring to the synthesis method of compound 1-4 in Example 4, 4 g of compound 1-3 and 1.89 g of phenol were used as raw materials to obtain compound 1-7 as a white powder, with a yield of 43.02% and a purity of 99.54%.

1H NMR (600MHz, DMSO-d6)δ2.63(CH2,m,2H), 2.66(CH2,m,2H), 3.01(CH3,s,3H), 3.13(CH2,m,2H), 3.16(CH3,m,3H), 3.67(CH2,t,2H), 4.50(CH,s,H), 7.26(CH,m,H), 7.29(2CH,m,2H), 7.42(2CH,m,2H)ppm. MS:m/z:375.16 (M+1).

### Example 8 Preparation of Compound 1-8

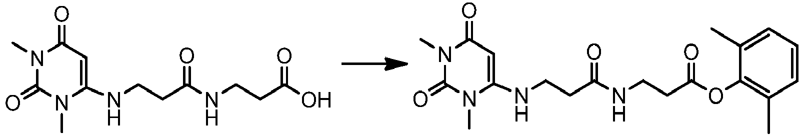

By By referring to the synthesis method of compound 1-4 in Example 4, 4 g of compound 1-3 and 2.46 g of 1.3-dimethylphenol were used as raw materials to obtain compound 1-8 as a white powder, with a yield of 45.74% and a purity of 99.14%.

1H NMR (600MHz, DMSO-d6)δ2.15(2CH3,s,6H), 2.63(CH2,m,2H), 2.66(CH2,m,2H), 3.01(CH3,s,3H), 3.13(CH2,m,2H), 3.16(CH3,m,3H), 3.67(CH2,t,2H), 4.50(CH,s,H), 7.01(2CH,d,2H), 7.24(CH,m,H)ppm. MS:m/z:403.19 (M+1).

### Example 9 Preparation of Compound 1-9

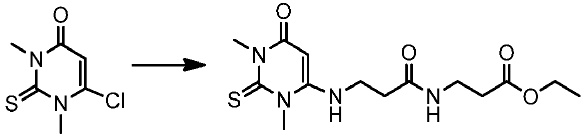

To a 100 mL single-necked flask was added 3 g of 1,3-dimethyl-6-chloro-2-thioxo-4(1H)-pyrimidinone (CAS:6972-27-6), 3.18 g of DIEA, 30 mL of isopropanol and 2.95 g of beta-alanine-β-alanine ethyl ester (CAS:754933-31- 8). After addition, the reaction was warmed to reflux and reacted under stirring. After stirring for 8h, the reaction stopped stirring and was separated by sand column chromatography to give compound 1-9 as a white powder, with a yield of 49.35% and a purity of 95.6%.

1H NMR (600 MHz, DMSO-d6)δ1.29(CH3,t,3H), 2.65(CH2,m,2H), 2.66(CH2,m,2H), 3.07(CH3,s,3H), 3.13(CH2,m,2H), 3.34(CH3,s,3H), 3.79(CH2,t,2H), 4.13(CH2,q,2H), 6.09(CH,s,H) ppm. MS:m/z:343.14 (M+1).

### Example 10 Preparation of Compound 1-10

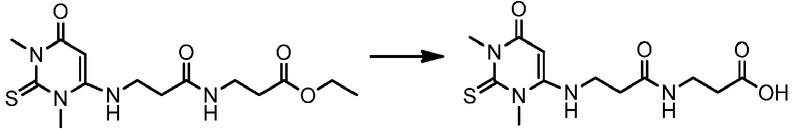

To a 100mL single-necked flask was added 1.3g of Compound 1-9, 0.23g of NaOH, 30mL of ethanol, and 3mL of water. After addition, the reaction was stirred at room temperature. After 2h of stirring, the reaction was sampled and monitored by TLC, which showed that the raw materials in the reaction solution were reacted completely after 2h of stirring. The reaction solution was then adjusted to pH to neutral with concentrated hydrochloric acid, concentrated to dryness directly, and then concentrated with ethanol for several times to obtain compound 1-10 as an off-white powder, with a yield of 77.26% and a purity of 99.12%.

1H NMR (600 MHz, DMSO-d6)δ2.49(CH2,t,2H), 2.66(CH2,t,2H), 3.07(CH3,m,3H), 3.13(CH2,m,2H), 3.34(CH3,s,3H), 3.67(CH2,t,2H), 6.09(CH,s,H) ppm. MS:m/z:315.10 (M+1).

### Example 11 Preparation of Compound 1-11

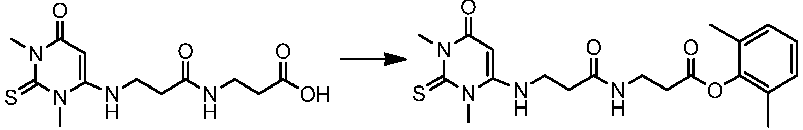

By By referring to the synthesis method of compound 1-4 in Example 4, 1.19 g of compound 1-10 and 0.69 g of 1,3-dimethylphenol were used as raw materials to obtain compound 1-11 as off-white powder, with a yield of 47.47% and a purity of 98.13%.

1H NMR (600MHz, DMSO-d6)δ2.15(2CH3,s,6H), 2.63(CH2,m,2H), 2.66(CH2,m,2H), 3.07(CH3,s,3H), 3.13(CH2,m,2H), 3.34(CH3,m,3H), 3.67(CH2,t,2H), 6.09(CH,s,H), 7.01(2CH,d,2H), 7.24(CH,m,H)ppm. MS:m/z:419.17 (M+1).

### Example 12 Preparation of Compound 1-12

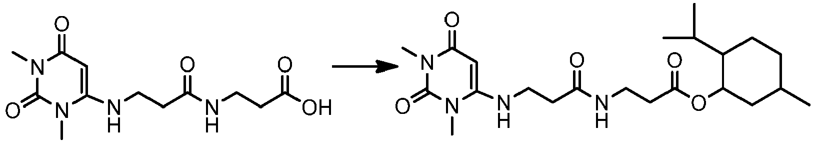

By By referring to the synthesis method of compound 1-4 in Example 4, 4 g of compound 1-3 and 3.14 g of menthol were used as raw materials to obtain compound 1-12 as a white powder, with a yield of 40.85% and a purity of 98.73%.

1H NMR (600MHz, DMSO-d6)δ0.91(2CH3,m,6H), 0.96(CH3,m,3H), 1.52;1.27(2CH2,m,4H), 1.61(CH,m,H), 1.76;1.51(CH2,m,2H), 1.82(CH,m,H), 2.01(CH,m,H), 3.90(CH,s,H), 2.51(CH2,t,2H), 2.66(CH2,t,2H), 3.01(CH3,s,3H), 3.13(CH2,m,2H), 3.16(CH3,m,3H), 3.79(CH2,m,2H), 4.50(CH,s,H)ppm. MS:m/z:437.27 (M+1).

### Example 13 Preparation of Compound 1-13

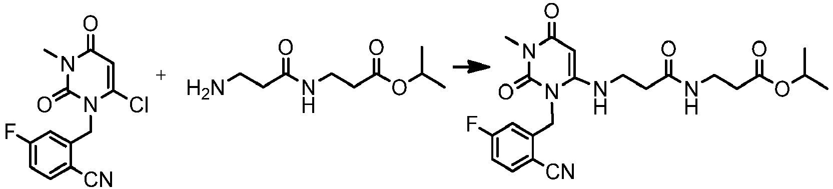

By referring to the synthesis method of Compound 1-1 in Example 1, 2-[(6-chloro-3,4-dihydro-3-methyl-2,4-dioxo-1(2H)-pyrimidinyl)methyl]-4-fluorobenzonitril e (generated by the reaction of 6-chloro-3-methyluracil and 2-cyano-5-fluorobenzyl bromide) and compound(CAS:1582394-73-7) were used as raw materials to obtain compound 1-13 as a white powder, with a yield of 47.36% and a purity of 99.48%.

1H NMR (600 MHz, DMSO-d6)δ1.32(2CH3,d,6H), 2.65(CH2,m,2H), 2.66(CH2,m,2H), 3.13(CH2,m,2H), 3.16(CH3,m,3H), 3.79(CH2,t,2H), 4.50(CH,s,H), 4.93(CH,m,H), 5.18(CH2,s,2H), 6.95(CH,m,H), 7.23(CH,m,H), 7.50(CH,m,H) ppm. MS:m/z:460.19 (M+1).

### Example 14 Preparation of Compound 1-14

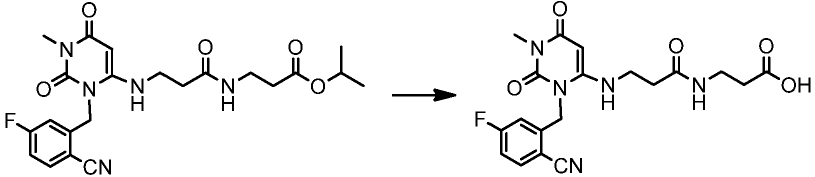

To a 100mL single-necked flask was added 7g of compound 1-13, 0.69g of sodium hydroxide and 70ml of anhydrous ethanol, and the reaction was stirred at room temperature for 3 hours. After the reaction was completed, the reaction was concentrated to a small amount of ethanol, added with water, then adjusted to pH = 5 with citric acid water, stirred for half an hour, and filtrated; the filter cake was pulped with water, filtrated, and vacuum-dried at 60 °C°C to obtain 3.5g of compound 1-14 as an off-white powder, with a yield of 90.26% and a purity of 99.31%.

1H NMR (600 MHz, DMSO-d6)δ2.49(CH2,m,2H), 2.66(CH2,m,2H), 3.13(CH2,m,2H), 3.16(CH3,m,3H), 3.67(CH2,t,2H), 4.50(CH,s,H), 5.18(CH2,s,2H), 6.95(CH,m,H), 7.23(CH,m,H), 7.50(CH,m,H) ppm. MS:m/z:418.14 (M+1).

### Example 15 Preparation of Compound 1-15

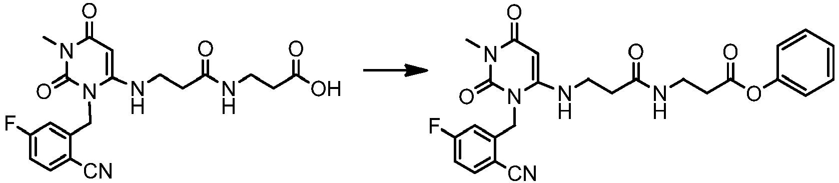

By By referring to the synthesis method of compound 1-4 in Example 4, 2 g of compound 1-14 and 0.5 g of phenol were uesd as raw materials to give compound 1-15 as a off-white powder, with a yield of 67.80% and a purity of 95.81%.

1H NMR (600MHz, DMSO-d6)δ2.63(CH2,m,2H), 2.66(CH2,m,2H), 3.13(CH2,m,2H), 3.16(CH3,m,3H), 3.67(CH2,t,2H), 4.50(CH,s,H), 5.18(CH2,s,2H), 6.95(CH,m,H), 7.23(CH,m,H), 7.26(CH,m,H), 7.29(2CH,m,2H), 7.42(2CH,m,2H), 7.50(CH,m,H) ppm. MS:m/z:494.18 (M+1).

### Example 16 Preparation of Compound 1-16

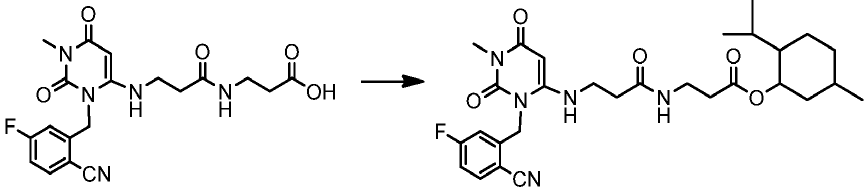

By By referring to the synthesis method of compound 1-4 in Example 4, 2 g of compound 1-14 and 0.82 g of menthol were used as raw materials to obtain compound 1-16 as a white powder, with a yield of 56.39% and a purity of 98.82%.

1H NMR (600MHz, DMSO-d6)δ0.91(2CH3,m,6H), 0.96(CH3,m,3H), 1.52;1.27(2CH2,m,4H), 1.61(CH,m,H), 1.76;1.51(CH2,m,2H), 1.82(CH,m,H), 2.01(CH,m,H), 2.51(CH2,t,2H), 2.66(CH2,t,2H), 3.13(CH2,m,2H), 3.16(CH3,m,3H), 3.79(CH2,m,2H), 3.90(CH,s,H), 4.50(CH,s,H), 5.18(CH2,s,2H), 6.95(CH,m,H), 7.23(CH,m,H), 7.50(CH,m,H) ppm. MS:m/z:556.29 (M+1).

### Example 17 Preparation of Compound 1-17

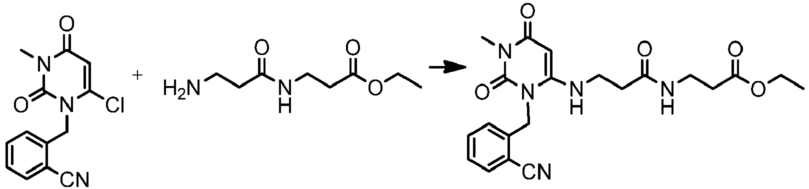

By By referring to the synthesis method of Compound 1-1 in Example 1, 2-[(6-chloro-3,4-dihydro-3-methyl-2,4-dioxo-1(2H)-pyrimidinyl)methyl]benzonitrile (generated by the reaction of 6-chloro-3-methyluracil and o-cyanobenzyl chloride), and β-alanine-β-alanine ethyl ester (CAS:754933- 31-8) were used as raw materials to obtain compound 1-17 as a white powder, with a yield of 49.36% and a purity of 98.89%.

1H NMR (600 MHz, DMSO-d6)δ1.29(CH3,t,3H), 2.65(CH2,m,2H), 2.66(CH2,m,2H), 3.13(CH2,m,2H), 3.16(CH3,m,3H), 3.79(CH2,t,2H), 4.13(CH2,q,2H), 4.50(CH,s,H), 5.18(CH2,s,2H), 7.41(CH,m,H), 7.44(CH,m,H), 7.52(CH,m,H), 7.61(CH,m,H)ppm. MS:m/z:428.19(M+1).

### Example 18 Preparation of Compound 1-18

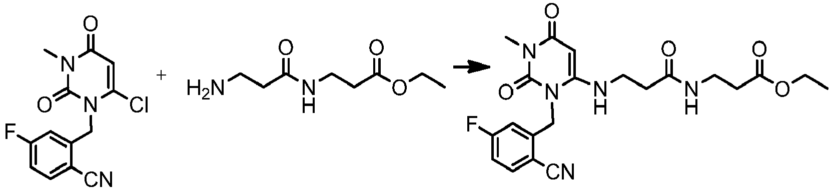

By By referring to the synthesis method of Compound 1-1 in Example 1, 2-[(6-chloro-3,4-dihydro-3-methyl-2,4-dioxo-1(2H)-pyrimidinyl)methyl]-4-fluorobenzonitril e and β-alanine- β-alanine ethyl ester (CAS: 754933-31-8) were used as the raw materials to obtain Compound 1- 18 as a white powder, with a yield of 48.08% and a purity of 99.46%.

1H NMR (600 MHz, DMSO-d6)δ1.29(CH3,t,3H), 2.65(CH2,m,2H), 2.66(CH2,m,2H), 3.13(CH2,m,2H), 3.16(CH3,m,3H), 3.79(CH2,t,2H), 4.13(CH2,q,2H), 4.50(CH,s,H), 5.18(CH2,s,2H), 6.95(CH,m,H), 7.23(CH,m,H), 7.50(CH,m,H) ppm. MS:m/z:446.18(M+1).

### Example 19 Preparation of Compound 1-19

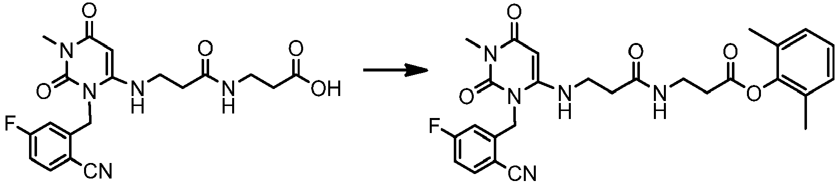

By By referring to the synthesis method of compound 1-4 in Example 4, 2 g of compound 1-14 and 0.64 g of 2,6-dimethylphenol were used as raw materials to obtain compound 1-19 as a white powder, with a yield of 52.00% and a purity of 99.23%.

1H NMR (600MHz, DMSO-d6)δ2.15(2CH3,s,6H), 2.63(CH2,m,2H), 2.66(CH2,m,2H), 3.01(CH3,s,3H), 3.13(CH2,m,2H), 3.16(CH3,m,3H), 3.67(CH2,t,2H), 4.50(CH,s,H), 5.18(CH2,s,2H), 6.95(CH,m,H), 7.01(2CH,m,2H), 7.23(CH,m,H), 7.24(CH,m,H), 7.50(CH,m,H) ppm. MS:m/z:522.21(M+1).

### Example 20 Preparation of Compound 1-20

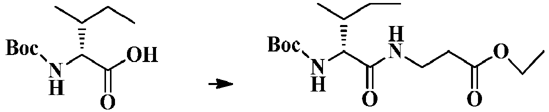

To a 1L single-necked flask was aded 31.1g of N-Boc-L-isoleucine, 27.2g of HOBT, and dissolved with 300 mL DCM. 20.4g of triethylamine and 38.6g of EDCI were added, after addition, the atmosphere of the reaction was replaced with AR gas three times. The reaction was stirred at 25°C for 1 hour, then added with 22.7g Beta-alanine ethyl ester hydrochloride, and stirred at 25 °C. The reaction was sampled and detected by TLC, and the raw material was completely reacted. The reaction solution was then washed with water for three times and 5% citric acid for four times (200mL*4), further washed with water and saturated sodium chloride, dried with anhydrous sodium sulfate, concentrated to dryness to obtain 42.6g of compound 1-20, as a oil, with a yield of 99.5%, which was directly used into the next step.

1H NMR (600MHz, DMSO-d6)δ0.90(CH3,t,3H), 1.11(CH3,d,3H), 1.29(CH3,t,3H), 1.38(3CH3,s,9H), 1.55(CH2,m,2H), 2.12(CH,m,H), 3.59(CH2,t,2H), 3.65(CH2,t,2H), 4.13(CH2,m,2H), 4.52(CH,d,H) ppm. MS:m/z:331.22 (M+1).

### Example 21 Preparation of Compound 1-21

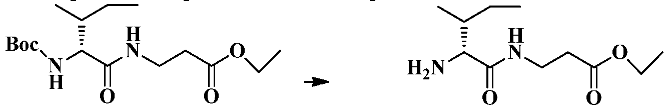

To a 500mL single-necked flask was added 42.5g of compound 1-20, and slowly added 150mL of 3M hydrochloric acid in ethanol to dissolve, and the reaction was stirred at 30°C. The reaction was sampled and detected by TLC, and the raw material was completely reacted. The reaction solution was then cooled to room temperature, and the pH was adjusted to about 5.0 with sodium bicarbonate. After filtration, the filtrate was dried with anhydrous sodium sulfate, concentrated to dryness to obtain 33g of Compound 1-21 as an oil, with a yield of nearly 100% and a purity of 99.34%.

1H NMR (600MHz, DMSO-d6)δ0.90(CH3,t,3H), 1.11(CH3,d,3H), 1.29(CH3,t,3H), 1.55(CH2,m,2H), 1.73(CH,m,H), 2.63(CH2,t,2H), 3.55(CH,m,H), 3.59(CH2,m,2H), 4.13(CH2,m,2H)ppm. MS:m/z:231.16 (M+1).

### Example 22 Preparation of Compound 1-22

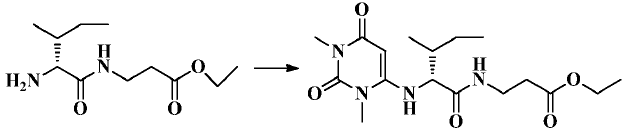

By By referring to the synthesis method of Compound 1-1 in Example 1, 1,3-dimethyl-6-chlorouracil and Compound 1-21 were used as raw materials to obtain Compound 1-22 as a white powder, with a yield of 62% and a purity of 99.97%.

1H NMR (600MHz, DMSO-d6)δ0.90(CH3,t,3H), 1.11(CH3,d,3H), 1.29(CH3,t,3H), 1.55(CH2,m,2H), 1.73(CH,m,H), 2.65(CH2,t,2H), 3.01(CH3,s,3H), 3.16(CH3,s,3H), 3.55(CH,m,H), 3.59(CH2,m,2H), 4.13(CH2,m,2H)ppm. MS:m/z:369.21 (M+1).

### Example 23 Preparation of Compound 1-23

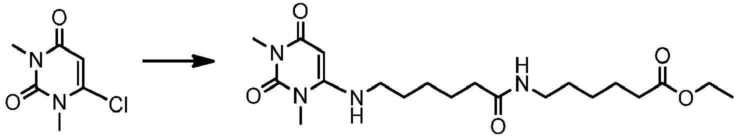

By By referring to the synthesis method of compound 1-1 in Example 1, 1,3-dimethyl-6-chlorouracil, and CAS: 16625-95-9 were used as raw materials to obtain compound 1-23 as a white powder, with a yield of 85.59% and a purity of 99.23%.

1H NMR (600MHz, DMSO-d6)δ1.28(CH2,m,2H), 1.29(2CH2,m,4H), 1.29(CH3,m,3H), 1.52(2CH2,m,4H), 1.64(CH2,m,2H), 2.13(CH2,m,2H), 2.32(CH2,t,2H), 2.87(CH2,t,2H), 3.01(CH3,m,3H), 3.02(CH2,m,2H), 3.16(CH3,s,3H), 4.13(CH2,q,2H), 4.50(CH,s,H)ppm. MS:m/z:411.25 (M+1).

### Example 24 Preparation of Compound 1-24

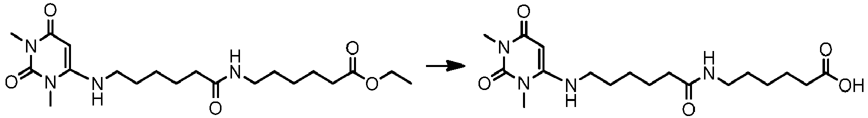

By By referring to the synthesis method of compound 1-3 in Example 3, compound 1-24 was prepared as a white powder, with a yield of 81.39% and a purity of 99.64%.

1H NMR (600MHz, DMSO-d6)δ1.28(CH2,m,2H), 1.29(2CH2,m,4H), 1.52(3CH2,m,6H), 2.13(CH2,m,2H), 2.30(CH2,t,2H), 2.87(CH2,t,2H), 3.01(CH3,m,3H), 3.02(CH2,m,2H), 3.16(CH3,s,3H), 4.50(CH,s,H)ppm. MS:m/z:383.22 (M+1).

### Example 25 Preparation of Compound 1-25

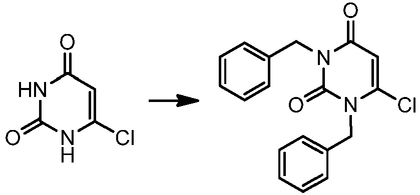

To a 250ml single-necked flask was added 10.0g 6-chlorouracil, 100.0g of DMSO, 23.8g of potassium carbonate, and 10.4g of benzyl chloride, and reacted at 20 °C until the raw materials were reacted completely, and stood overnight. The reaction solution was then diluted with 100 ml of water, and extracted with EA twice, with 200 ml each time; the extracted phase was combined, and washed with 100 ml of saturated saline; the organic phase was separated, concentrated under reduced pressure at 50 °C, -0.09MPa to obtain a yellow oily product, which was separated by column chromatography with a elution of P:E = 10: 1; then the product-containing liquid (50 °C, -0.09Mpa) was concentrated to obtain compound 1-25 as a white powder, with a yield of 72% and a purity of 99.12%.

1H NMR (600MHz, DMSO-d6)δ5.18(2CH2,s,4H), 5.94(CH,s,H), 7.23(4CH,m,4H), 7.26(2CH,m,2H), 7.33(4CH,t,4H)ppm. MS:m/z:327.08 (M+1).

### Example 26 Preparation of Compound 1-26

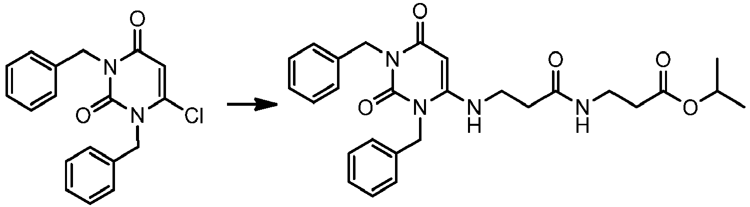

To a 100 ml single-necked flask was added 3.60 g of compound 1-25, 40.0 g of isopropanol, 4.63 g of triethylamine, and 4.62 g of compound (CAS: 1582394-73-7). The reaction was carried out at 85 °C, concentrated under reduced pressure at 55 °C, -0.09 MPa, then dissolved with 50 ml of saturated sodium bicarbonate, and extracted twice with 50 ml of dichloromethane for each time; the extracted phases were combined, concentrated under reduced pressure at 50°C, -0.09MPa to obtain 6.8g of white solid, which was then separated by column chromatography with a mixed solvent of dichloromethane: isopropanol=20:1 as elution; the product-containing liquid was concentrated (50°C, -0.09MPa) to obatin compound 1-26 as a white powder, with a yield of 68% and the purity of 99.88%.

1H NMR (600 MHz, DMSO-d6)δ1.32(2CH3,d,6H), 2.65(CH2,m,2H), 2.66(CH2,m,2H), 3.13(CH2,t,2H), 3.79(CH2,t,2H), 4.50(CH,s,H), 4.93(CH,m,H), 5.18(2CH2,s,4H), 7.23(4CH,m,4H), 7.26(2CH,m,2H), 7.33(4CH,t,4H) ppm. MS:m/z:493.24 (M+1).

### Example 27 Preparation of Compound 1-27

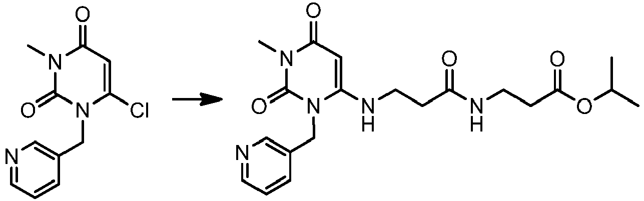

By By referring to the synthesis method of Compound 1-1 in Example 1, the indicated raw material (generated by the reaction of 6-chloro-3-methyluracil and 3-(chloromethyl)pyridine hydrochloride) and compound (CAS:1582394-73-7) were used as raw materials to obtain Compound 1-27 as a white powder, with a yield of 57% and a purity of 99.28%.

1H NMR (600 MHz, DMSO-d6)δ1.32(2CH3,d,6H), 2.65(CH2,m,2H), 2.66(CH2,m,2H), 3.13(CH2,t,2H), 3.16(CH3,m,3H), 3.79(CH2,t,2H), 4.50(CH,s,H), 4.93(CH,m,H), 5.18(CH2,s,2H), 7.38(CH,m,H), 7.86(CH,m,H), 8.45(CH,m,H), 8.59(CH,m,H)ppm. MS:m/z:418.20 (M+1).

### Example 28 Preparation of Compound 1-28

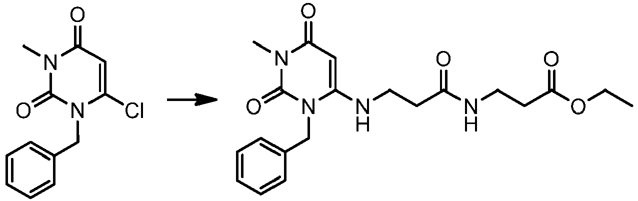

By By referring to the synthesis method of Compound 1-1 in Example 1, 1-benzyl-6-chloro-3-methyl-1H-pyrimidin-2,4-dione (generated by the reaction of 6-chloro-3-methyluracil benzyl chloride) and compound (CAS:754933-31-8) were used as raw materials to obtain Compound 1-28 as an off-white powder, with a yield of 55.3% and a purity of 98.75%.

1H NMR (600 MHz, DMSO-d6)δ1.29(CH3,d,3H), 2.65(CH2,m,2H), 2.66(CH2,m,2H), 3.13(CH2,t,2H), 3.16(CH3,m,3H), 3.79(CH2,t,2H), 4.13(CH2,t,2H), 4.50(CH,s,H), 5.18(CH2,s,2H), 7.23(2CH,m,2H), 7.26(CH,m,H), 7.33(2CH,m,2H)ppm. MS:m/z:403.19(M+1).

### Example 29 Preparation of Compound 1-29

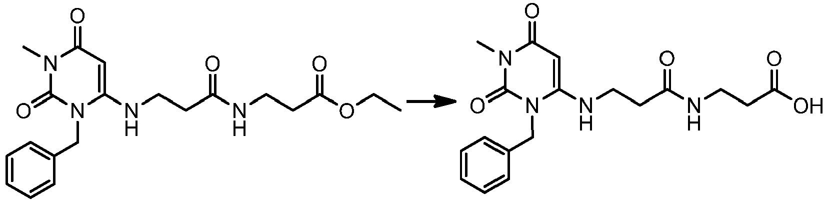

By By referring to the synthesis method of compound 1-3 in Example 3, compound 1-28 was used as raw material to obtain compound 1-29 as an off-white powder, with a yield of 73.5% and a purity of 99.32%.

1H NMR (600 MHz, DMSO-d6)δ2.49(CH2,t,2H), 2.66(CH2,t,2H), 3.13(CH2,m,2H), 3.16(CH3,m,3H), 3.67(CH2,t,2H), 4.50(CH,s,H), 5.18(CH2,s,2H), 7.23(2CH,m,2H), 7.26(CH,m,H), 7.33(2CH,m,2H)ppm. MS:m/z:375.16(M+1).

### Example 30 Preparation of Compound 1-30

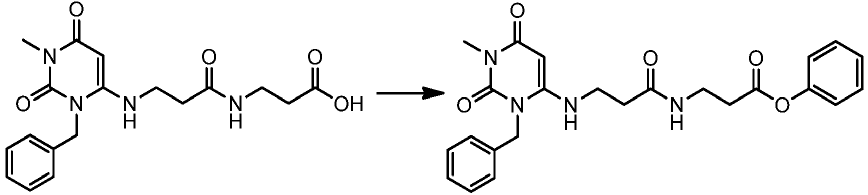

By By referring to the synthesis method of compound 1-4 in Example 4, 8.2 g of compound 1-29 and 2.3 g of phenol were used as raw materials to obtain compound 1-30 as an off-white powder, with a yield of 59.3% and a purity of 98.98%.

1H NMR (600 MHz, DMSO-d6)δ2.63(CH2,m,2H), 2.66(CH2,m,2H), 3.13(CH2,m,2H), 3.16(CH3,m,3H), 3.67(CH2,t,2H), 4.50(CH,s,H), 5.18(CH2,s,2H), 7.23(2CH,m,2H), 7.26(2CH,m,2H), 7.29(2CH,m,2H), 7.33(2CH,m,2H), 7.42(2CH,m,2H)ppm. MS:m/z:451.19(M+1).

### Example 31 Preparation of Compound 1-31

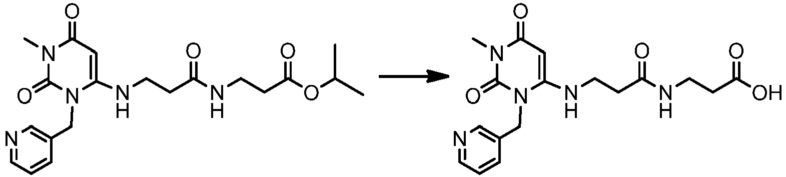

By By referring to the synthesis method of compound 1-3 in Example 3, compound 1-27 was used as raw material to obtain compound 1-31 as an off-white powder, with a yield of 76.8% and a purity of 98.67%.

1H NMR (600 MHz, DMSO-d6)δ2.49(CH2,t,2H), 2.66(CH2,t,2H), 3.13(CH2,m,2H), 3.16(CH3,m,3H), 3.67(CH2,t,2H), 4.50(CH,s,H), 5.18(CH2,s,2H), 7.38(CH,t,H), 7.86(CH,m,H), 8.45(CH,m,H), 8.59(CH,m,H)ppm. MS:m/z:376.15 (M+1).

### Example 32 Preparation of Compound 1-32

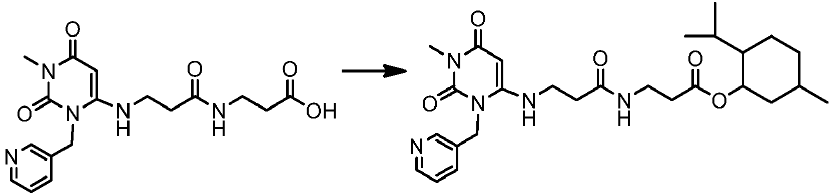

By By referring to the synthesis method of compound 1-4 in Example 4, 1 g of compound 1-31 and 1 g of menthol were used as raw materials to obtain compound 1-32 as an off-white powder, with a yield of 51% and a purity of 98.27%.

1H NMR (600 MHz, DMSO-d6)δ0.91(2CH3,m,6H), 0.96(CH3,m,3H), 1.52;1.27(2CH2,m,4H), 1.61(CH,m,H), 1.76;1.51(CH2,m,2H), 1.82(CH,m,H), 2.01(CH,m,H), 2.51(CH2,t,2H), 2.66(CH2,t,2H), 3.13(CH2,m,2H), 3.16(CH3,m,3H), 3.79(CH2,m,2H), 3.90(CH,m,H), 4.50(CH,s,H), 5.18(CH2,s,2H), 7.38(CH,t,H), 7.86(CH,m,H), 8.45(CH,m,H), 8.59(CH,m,H)ppm. MS:m/z:514.30 (M+1).

### Example 33 Preparation of Compound 1-33

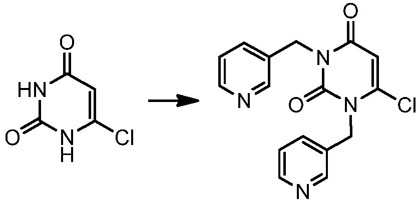

To a 250 ml single-necked flask was added 6 g of 6-chlorouracil, 60 g of DMSO, 6.7 g of chloromethyl pyridine hydrochloride and 11.6 g of potassium carbonate, and the reaction was carried out at 15 °C under stirring overnight. The reaction was then stopped; the reaction solution was diluted with 100 ml of water, and extracted with EA twice with 100 ml for each time; after extraction, the extracted phases were combined, and washed with 100 ml of saturated saline; the organic phase was separated, concentrated under reduced pressure at 50 °C, -0.09MPa to obtain compound 1-33 as a yellow oil, which was directly used in the next reaction step, with a yield of 62% and a purity of 98.35%.

1H NMR (600MHz, DMSO-d6)δ5.18(2CH2,s,4H), 5.94(CH,s,H), 7.38(2CH,m,2H), 7.86(2CH,m,2H), 8.45(2CH,t,2H), 8.592CH,t,2H)ppm. MS:m/z:329.07 (M+1).

### Example 34 Preparation of Compound 1-34

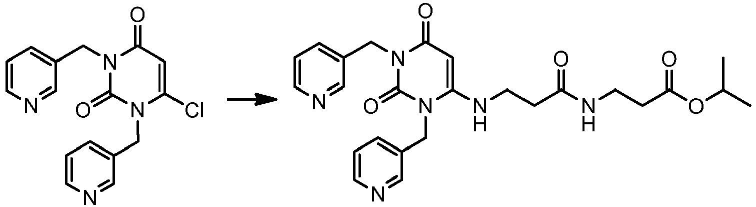

By By referring to synthesis method of compound 1-26 in Example 26, 2.3 g of Compound 1-33 and 2.94 g of Compound (CAS: 1582394-73-7) were used as raw materials to obtain Compound 1-34 as a light yellow powder, with a yield of 46% and a purity of 99.75%.

1H NMR (600MHz, DMSO-d6)δ1.32(2CH3,d,6H), 2.65(CH2,m,2H), 2.66(CH2,m,2H), 3.13(CH2,t,2H), 3.79(CH2,t,2H), 4.50(CH,s,H), 4.93(CH,m,H), 5.18(2CH2,s,4H), 7.38(2CH,m,2H), 7.86(2CH,m,2H), 8.45(2CH,t,2H), 8.59(2CH,t,2H) ppm. MS:m/z:495.23 (M+1).

### Example 35 Preparation of Compound 1-35

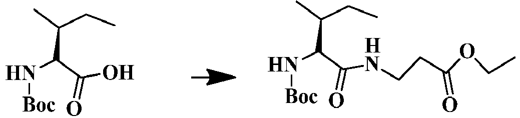

By referring to the synthesis method of compound 1-20 in Example 20, 5.3 g of N-tert-butoxycarbonyl-D-isoleucine, 3.9 g of Beta-alanine ethyl ester hydrochloride, 3.5 g of triethylamine, 4.7 g of HOBT and 6.6 g of EDCI were used as raw materials to obtain compound 1-34 as a light yellow oil, with a yield of 95% and a purity of 99.36%.

1H NMR (600MHz, DMSO-d6)δ0.90(CH3,t,3H), 1.11(CH3,d,3H), 1.29(CH3,t,3H), 1.38(3CH3,s,9H), 1.55(CH2,m,2H), 2.12(CH,m,H), 3.59(CH2,t,2H), 3.65(CH2,t,2H), 4.13(CH2,m,2H), 4.52(CH,d,H) ppm. MS:m/z:331.22 (M+1).

### Example 36 Preparation of Compound 1-36

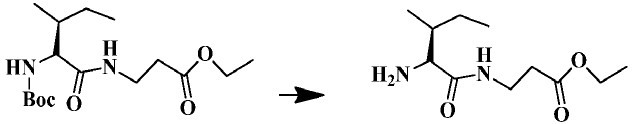

By By referring to the synthesis method of Compound 1-21 in Example 21, 6.9 g of Compound 1-35 and 2 mL of a solution of 3M hydrochloric acid in ethanol were used to react to obtain Compound 1-36 as a light yellow oil, with a yield of 100% and a purity of 99.40%.

1H NMR (600MHz, DMSO-d6)δ0.90(CH3,t,3H), 1.11(CH3,d,3H), 1.29(CH3,t,3H), 1.55(CH2,m,2H), 1.73(CH,m,H), 2.63(CH2,t,2H), 3.55(CH,m,H), 3.59(CH2,m,2H), 4.13(CH2,m,2H)ppm. MS:m/z:231.16 (M+1).

### Example 37 Preparation of Compound 1-37

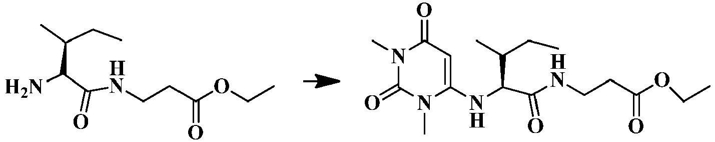

By referring to the synthesis method of Compound 1-1 in Example 1, 3.8 g of 1,3-dimethyl-6-chlorouracil and 5 g of Compound 1-36 were used as raw materials to obtain Compound 1-37 as light yellow powder, with a yield of 55% and a purity of 99.62%.

1H NMR (600MHz, DMSO-d6)δ0.90(CH3,t,3H), 1.11(CH3,d,3H), 1.29(CH3,t,3H), 1.55(CH2,m,2H), 1.73(CH,m,H), 2.65(CH2,t,2H), 3.01(CH3,s,3H), 3.16(CH3,s,3H), 3.55(CH,m,H), 3.59(CH2,m,2H), 4.13(CH2,m,2H)ppm. MS:m/z:369.21 (M+1).

### Example 38 Preparation of Compound 1-38

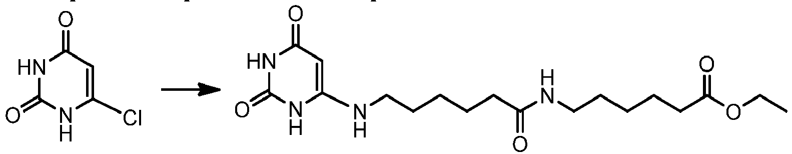

By referring to the synthesis method of Compound 1-1 in Example 1, 6-chlorouracil, and CAS:16625-95-9 were used as raw materials to obtain Compound 1-38 as a off-white powder, with a yield of 65% and a purity of 98.37%.

1H NMR (600MHz, DMSO-d6)δ1.28(CH2,m,2H), 1.29(2CH2,m,4H), 1.29(CH3,m,3H), 1.52(2CH2,m,4H), 1.64(CH2,m,2H), 2.13(CH2,m,2H), 2.32(CH2,t,2H), 2.87(CH2,t,2H), 3.02(CH2,t,2H), 4.13(CH2,q,2H), 4.50(CH,s,H)ppm. MS:m/z:383.22 (M+1).

### Example 39 Preparation of Compound 1-39

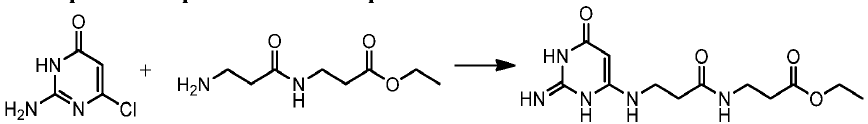

By referring to the synthesis method of Compound 1-1 in Example 1, 2-amino-6-chloro-4-hydroxypyrimidine and β-alanine-β-alanine ethyl ester (CAS: 754933-31-8) were used as raw materials to obtain Compound 1-2 as an off-white powder, with a yield of 47.8% and a purity of 98.6%.

1H NMR (600 MHz, DMSO-d6)δ1.29(CH3,t,3H), 2.65(CH2,m,2H), 2.66(CH2,m,2H), 3.13(CH2,t,2H), 3.79(CH2,t,2H), 4.13(CH2,q,2H), 6.09(CH,s,H)ppm. MS:m/z:298.14(M+1).

### Example 40 Preparation of Compound 1-40

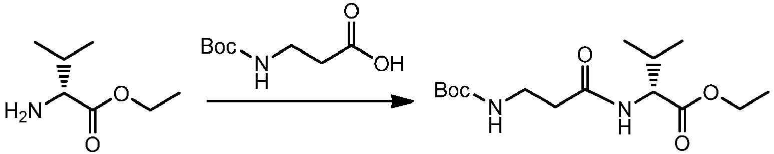

To a 250mL single-necked flask was added 12g of ethyl L-valinate hydrochloride, then added 15g of N-BOC-alanine, 17g of EDCI, 30g of triethylamine, 15g of HOBT, and 150mL of DCM, and the reaction was stirred at room temperature overnight. The reaction was sampled and monitored by TLC until the completion of the reaction. The reaction was then stopped, and washed with water, saturated sodium bicarbonate solution, saturated citric acid, and saturated sodium chloride solution, and then dried with anhydrous sodium sulfate; the organic phase was concentrated to dryness to obtain compound 1-40 as a colorless oil, with a yield of 76.49% and a purity of 98.6%.

1H NMR (600MHz, DMSO-d6)δ0.91(2CH3,d,6H), 1.29(CH3,t,3H), 1.38(3CH3,s,9H), 2.21(CH,m,H), 2.44(CH2,t,2H), 3.44(CH2,t,2H), 4.21(CH2,m,2H), 4.21(CH,m,H)ppm. MS:m/z:317.20 (M+1).

### Example 41 Preparation of Compound 1-41

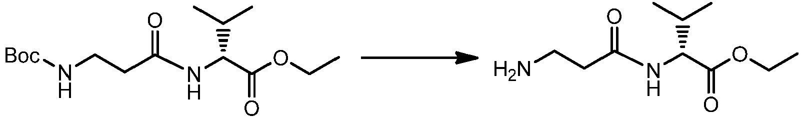

To a 250mL single-necked flask was added 15g of Compound 1-40, then added 100mL of anhydrous ethanol, and 25mL of 11M hydrochloride in ethanol. The reaction was stirred at room temperature overnight and monitored until the reaction was complete, after which the reaction was adjusted to pH about 5.0 with sodium bicarbonate, and filtered; the filtrate was dried over anhydrous sodium sulfate and concentrated to dryness to give compound 1-41 as a colorless oil, with a yield of 97.53% and a purity of 98.49%.

1H NMR (600MHz, DMSO-d6)δ0.91(2CH3,d,6H), 1.29(CH3,t,3H), 2.21(CH,m,H), 2.44(CH2,m,2H), 2.93(CH2,t,2H), 4.21(CH2,m,2H), 4.21(CH,m,H)ppm. MS:m/z:217.15 (M+1).

### Example 42 Preparation of Compound 1-42

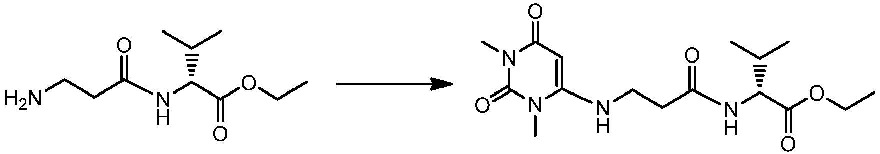

By referring to the synthesis method of Compound 1-1 in Example 1, 8.07 g of 1,3-dimethyl-6-chlorouracil and 10 g of Compound 1-41 were used as raw materials to obtain Compound 1-42 as a white powder, with a yield of 42.7% and a purity of 98.04%.

1H NMR (600MHz, DMSO-d6)δ0.91(2CH3,d,6H), 1.29(CH3,t,3H), 2.21(CH,m,H), 2.44(CH2,t,2H), 3.01(CH3,s,3H), 3.13(CH2,m,2H), 3.16(CH3,m,3H), 4.21(CH2,m,2H), 4.21(CH,m,H), 4.50(CH,s,H)ppm. MS:m/z:355.19 (M+1).

### Example 43 Preparation of Compound 1-43

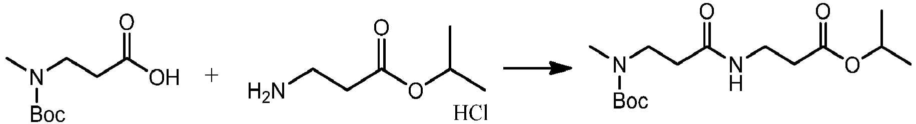

To a 250mL round-bottomed flask was added 8.92g of 3-[(tert-butoxycarbonyl)(methyl)amino]propanoic acid, 8.8g of β-alanine isopropyl ester hydrochloride, and 100mL of dichloromethane, and stirred to dissolve until clarified, and then cooled down to 0 °C. 23mL of triethylamine was measured and added slowly dropwise to the round-bottomed flask. After dropwise addition, the reaction system was added with 15.15g of EDCI, warmed to 40°C and stirred overnight. At the end of the reaction, the reaction solution was added with 30 ml of water, and stirred, and the organic layer was separated. The organic layer was washed twice with 30 ml of water and then dried with anhydrous sodium sulfate. after filtration, the filtrate was collected and concentrated to dryness under reduced pressure to give compound 1-43 as a colorless oil, with a yield of 67.9% and a purity of 98.43%.

1H NMR (600MHz, DMSO-d6)δ1.32(2CH3,d,6H), 1.38(3CH3,s,9H), 2.65(CH2,m,2H), 2.66(CH2,m,2H), 3.24(CH2,t,2H), 3.47(CH3,s,3H), 3.79(CH2,t,2H), 4.93(CH,m,H)ppm. MS:m/z:317.20 (M+1).

### Example 44 Preparation of Compound 1-44

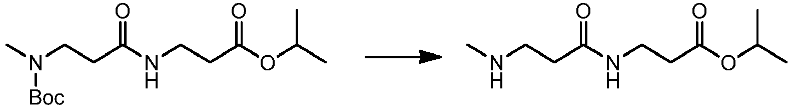

To a reaction flask was added 40mL of DCM, and 11.1g of compound 1-43, and 20mL of trifluoroacetic acid was added dropwise, and the reaction was stirred at room temperature. The reaction solution was concentrated to dryness to obtain an oily substance, then dissolved with 30mL of DCM, and continued to concentrate to dryness. After concentration, the concentrate was pulped with 50mL of ethyl acetate and concentrated to dryness under reduced pressure to obtain compound 1-44 as colorless oil, with a yield of 91.6% and a purity of 98.74%.

1H NMR (600MHz, DMSO-d6)δ1.32(2CH3,d,6H), 2.52(CH2,t,2H), 2.65(CH2,t,2H), 2.83(CH2,t,2H), 3.26(CH3,s,3H), 3.79(CH2,t,2H), 4.93(CH,m,H)ppm. MS:m/z:217.15 (M+1).

### Example 45 Preparation of Compound 1-45

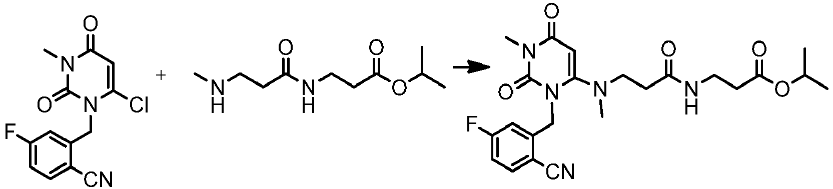

By referring to the synthesis method of Compound 1-1 in Example 1, 6 g of 2-[(6-chloro-3,4-dihydro-3-methyl-2,4-dioxo-1(2H)-pyrimidinyl)methyl]-4-fluorobenzonitril e (generated by the reaction of 6-chloro-3-methyluracil and 2-cyano-5-fluorobenzyl bromide) and 6.07 g of Compound 1-44 were used as raw materials to obtain Compound 1-13, as a white powder, with a yield of 48.45% and a purity of 98.76%.

1H NMR (600MHz, DMSO-d6)δ1.32(2CH3,d,6H), 2.52(CH2,t,2H), 2.65(CH2,t,2H), 2.83(CH2,t,2H), 3.04(CH3,s,3H), 3.16(CH3,s,3H), 3.79(CH2,t,2H), 4.50(CH,s,H), 4.93(CH,m,H), 5.18(CH2,s,2H), 6.95(CH,m,H), 7.23(CH,m,H), 7.50(CH,m,H)ppm. MS:m/z:474.21 (M+1).

### Example 46 Preparation of Compound 1-46

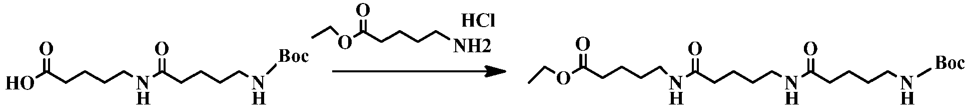

To a 500 mL single-necked flask was added 21 g of 5-(6-(tert-butoxycarbonylamino)hexylamino)pentanoic acid, 13.4 g of triethylamine, and 300 mL of DCM and stirred. The atmosphere of the reaction was replaced with Ar gas three times. 19.0 g of EDCI was added at 10 °C. After addition, the reaction was stirred at room temperature for 1 hour. 15.3g of ethyl 5-aminopentanoate hydrochloride was added. After addition, the reaction was stirred overnight at room temperature. After completion of the reaction, the reaction solution was washed with water (50mL*2 times), and 5% citric acid solution (50mL*2 times); the combined aqueous phase was extracted once with 200 mLof DCM; the organic phase was combined and washed once with water and saturated sodium chloride, dried with anhydrous sodium sulfate, concentrated to dryness to obtain compound 1-46 as a colorless oil, with a yield of 70% and a purity of 98.43%.

1H NMR (600MHz, DMSO-d6)δ1.29(CH3,t,3H), 1.38(3CH3,s,9H), 1.52(3CH2,m,6H), 1.53(2CH2,m,4H), 1.64(CH2,m,2H), 2.13(2CH2,m,4H), 2.32(CH2,t,2H), 3.02(2CH2,t,4H), 3.18(CH2,m,2H), 4.13(CH2,m,2H)ppm. MS:m/z:444.30 (M+1).

### Example 46 Preparation of Compound 1-47

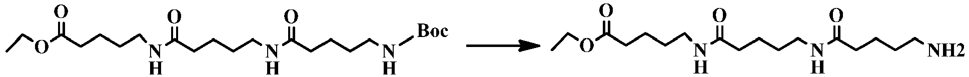

To a 250mL single-necked flask was added 20.0g of compound 1-46, and 50mL of ethanol hydrochloride (3M). The reaction was stirred and carried out at room temperature, then sampled and detected by TLC, and the raw material was completely reacted, after which the reaction solution was adjusted to pH about 5.0 with sodium bicarbonate. After filtration, the filtrate was dried with anhydrous sodium sulfate and concentrated to dryness to obtain compound 1-47 as a colorless oil, with a yield of nearly 100% and a purity of 98.23%.

1H NMR (600MHz, DMSO-d6)δ1.29(CH3,t,3H), 1.52(3CH2,m,6H), 1.53(2CH2,m,4H), 1.64(CH2,m,2H), 2.13(2CH2,m,4H), 2.32(CH2,t,2H), 2.65(CH2,m,2H), 3.02(2CH2,t,4H), 4.13(CH2,m,2H)ppm. MS:m/z:344.25 (M+1).

### Example 47 Preparation of Compound 1-48

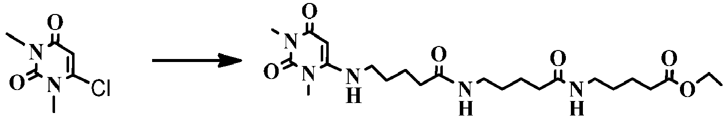

By referring to the synthesis method of Compound 1-1 in Example 1, 2.7 g of 1,3-dimethyl-6-chlorouracil and 6.2 g of Compound 1-47 were used as raw materials to obtain Compound 1-48 as a pink powder, with a yield of 41.42% and a purity of 99.38%.

1H NMR (600MHz, DMSO-d6)δ1.29(CH3,t,3H), 1.52(3CH2,m,6H), 1.53(2CH2,m,4H), 1.64(CH2,m,2H), 2.13(2CH2,m,4H), 2.32(CH2,t,2H), 2.87(CH2,m,2H), 3.01(CH3,m,3H), 3.02(2CH2,m,4H), 3.16(CH3,s,3H), 4.13(CH2,m,2H), 4.50(CH,s,H)ppm. MS:m/z:482.29 (M+1).

### Example 49 Preparation of Compound 1-49

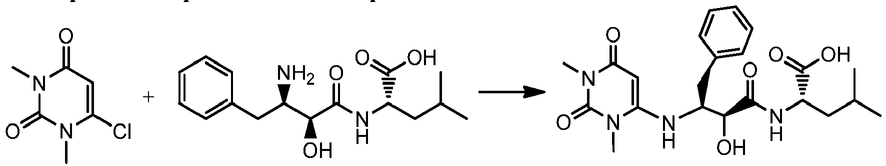

By referring to the synthesis method of compound 1-1 in Example 1, 1,3-dimethyl-6-chlorouracil and Ubenimex (CAS: 58970-76-6) were used as raw materials to obtain compound 1-49 as a white powder, with a yield of 51.63% and a purity of 98.46%.

1H NMR (600MHz, DMSO-d6)δ0.91(2CH3,d,6H), 1.49(CH,m,H), 1.75(CH2,t,2H), 2.92; 2.67(CH2,m,2H), 3.01(CH3,s,3H), 3.16(CH3,s,3H), 3.31(CH,m,H), 4.39(CH,d,H), 4.50(CH,m,H), 4.55(CH,m,H), 7.27(CH,m,H), 7.29(2CH,m,2H), 7.40(2CH,m,2H)ppm. MS:m/z:447.22 (M+1).

### Example 50 Preparation of Compound 1-50

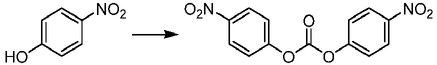

20 g of 4-nitrophenol was weighed and dissolved in 200 mL of DCM, 21 g of DIPEA was added, and the reaction was transferred to an ice-water bath to cool to 0°C, the atmosphere of the reaction was replaced with N₂ three times. The temperature was lowered to 0°C and 25 g of triphosgene (dissolved in 100 mL DCM) was added dropwise to the reaction solution. After dropping, the reaction was stirred at room temperature and monitored by TLC until the raw material was almost completely reacted and the reaction stopped. The reaction solution was washed repeatedly with 40 mL of water until nearly neutral, then washed with 50 mL of saturated saline, dried with anhydrous sodium sulfate, and filtered. The filtrate was concentrated under reduced pressure at 45°C to dryness to give 22 g of compound 1-50 as an off-white powder, with a yield of 100%.

1HNMR (600 MHz, DMSO-d6)δ7.52(4CH,m,4H), 8.3(4CH,m,4H)ppm. MS:m/z:305.03 (M+1).

### Example 51 Preparation of Compound 1-51

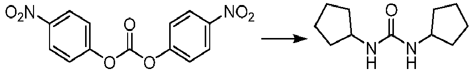

22 g of compound 1-50 was weighed and dissolved in 250 mL of DCM, 13 g of cyclopentylamine was added, the atmosphere of the reaction was replaced with N₂ three times, and the reaction was transferred to an oil bath and heated to 40 °C with stirring. The temperature was raised to 40 °C and 25 g of triphosgene (dissolved in 100 mL of DCM) was added dropwise to the reaction solution. TLC monitored until the raw material was almost completely reacted and the reaction stopped. The reaction solution was then concentrated under reduced pressure at 45°C to dryness, and the concentrate was added with 150mL of methanol and heated to dissolve, after dissolved to be clear, added with 100mL of water and stirred at room temperature. After filtration, the filter cake was vacuum-dried in an oven at 60 °C to give 8 g of compound 1-51 as a gray-brown powder, with a yield of 56.4%.

1H NMR (600 MHz, DMSO-d6)δ 1.73;1.63(4CH2,m,8H), 1.85;1.60(4CH2,m,8H), 3.61(2CH,m,2H)ppm. MS:m/z:197.16 (M+1).

### Example 52 Preparation of Compound 1-52

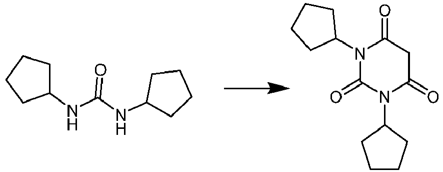

8 g of Compound 1-51 was weighed and dissolved in 80 mL of DCM, the atmosphere of the reaction was replaced with N₂ three times and 5.6 g of malonyl chloride was added dropwise. After dropwise addition, the reaction was transferred to an oil bath and heated to 45°C with stirring. The temperature was raised to 45°C and the reaction was kept at the temperature and carried out under stirringd. TLC monitored until the raw material was almost completely reacted and the reaction was stopped. The reaction solution was concentrated to dryness under reduced pressure at 45°C to give a dark green solid, which was then prepared and purified by column chromatography (PE:EA=15:1-8:1 gradient), and the main component was eluted and collected, concentrated to dryness to give 7g of compound 1-52, as a yellow powder, with a yield of 65%.

1H NMR (600 MHz, DMSO-d6)δ 1.73;1.63(4CH2,m,8H), 1.85;1.60(4CH2,m,8H), 3.07(CH2,s,2H), 3.61(2CH,m,2H)ppm. MS:m/z:265.15 (M+1).

### Example 53 Preparation of Compound 1-53

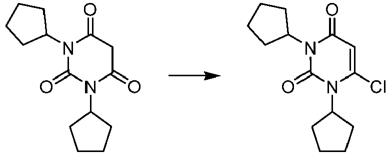

7.0 g of Compound 1-52 and 3.9 g of N,N-diethylaniline were weighed and 35 mL of phosphorus oxychloride was added, the atmosphere of the reaction was replaced with N₂ three times, and the reaction was transferred to an oil bath and heated to 100 °C with stirring. The temperature was raised to 100°C and the reaction was kept at the temperature and carried out under stirring. TLC monitored until the raw material was almost completely reacted and the reaction stopped. The reaction solution was slowly added to 300 mL of ice water, stirred thoroughly, and then extracted with 300 mL of DCM for three times; the organic phase was washed successively with 100 mL of water and 100 mL of saturated saline for two times, dried with anhydrous sodium sulfate and filtered. The filtrate was concentrated at 45°C under reduced pressure to dryness to give brown oil, which was then prepared and purified by column chromatography (PE:EA=50:1-20:1 gradient), and the main component was eluted and collected, concentrated to dryness to give 5.3 g of compound 1-53 as a yellow oil, with a yield of 70.09%.

1H NMR (600 MHz, DMSO-d6)δ 1.73;1.63(4CH2,m,8H), 1.85;1.60(4CH2,m,8H), 3.61(2CH,m,2H), 5.65(CH,s,H)ppm. MS:m/z:283.11 (M+1).

### Example 54 Preparation of Compound 1-54

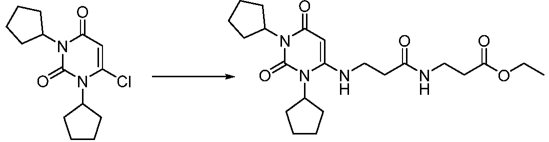

1.0 g of Compound 1-53 and 0.7 g of β-alanine-β-alanine ethyl ester (CAS: 754933-31-8) were weighed and added to 10 mL of isopropanol and stirred to dissolve, and then 1.4 g of DIPEA was added. The temperature was raised to 85°C and and the reaction was kept at the temperature and reacted for 4 h under stirring, and then stirred at room temperature overnight. TLC monitored until the raw material was completely reacted and the reaction stopped. The reaction solution was concentrated at 55°C to dryness, and then dissolved by adding 200 mL of DCM, then washed successively with 40 mL of water and 40 mL of saturated saline, dried with anhydrous sodium sulfate, and filtered; the filtrate was concentrated to dryness to obtain a oily substance, which was then prepared and purified by column chromatography (methanol:DCM= 1: 100-4: 100, gradient), and the main component was eluted and collected, concentrated to dryness to obtain 0.73 g of compound 1-54 as a light yellow powder, with a yield of 47.4% and a purity of 98.77%.

1H NMR (600MHz, DMSO-d6)δ1.07(CH3,t,3H), 1.73;1.63(4CH2,m,8H), 1.85;1.60(4CH2,m,8H), 2.64(CH2,m,2H), 2.65(CH2,m,2H), 3.61(2CH,m,2H), 3.61(CH2,m,2H), 3.79(CH2,t,2H), 4.01(CH2,m,2H), 4.21(CH,s,H)ppm. MS:m/z:435.25 (M+1).

### Example 55 Preparation of Compound 1-55

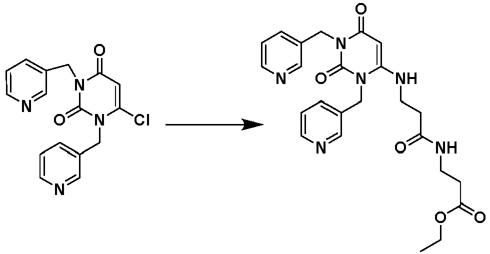

10.0 g of compound 1-33, 5.7 g of β-alanine-β-alanine ethyl ester (CAS: 754933-31-8), and 9.1 g of triethylamine were weighed and dissolved in 100 mL of ethanol, and the reaction was stirred at 80 °C. The reaction was then stirred at room temperature overnight and monitored by TLC until the raw material was completely reacted and the reaction stopped. The reaction solution was concentrated to dryness at 50°C, and extracted with DCM; the organic phase was washed with water and saturated sodium chloride, dried with anhydrous sodium sulfate, and concentrated to dryness, which was then prepared and purified by column chromatography (DCM:EtOH=100:1+2% ammonia), and the main component was eluted and collected, concentrated to dryness to obtain 8.2 g of a yellow solid, which was recrystallized by adding 30 mL of anhydrous ethanol, and after filtration, the filter cake was dried to give 7.6 g of Compound 1-55 as white powder, with a yield of 52% and a purity of 98.55%.

1H NMR (600MHz, DMSO-d6)δ1.07(CH3,t,3H), 2.64(CH2,m,2H), 2.65(CH2,m,2H), 3.61(CH2,t,2H), 3.79(CH2,t,2H), 4.01(CH2,m,2H), 4.21(CH,s,H), 4.48(2CH2,s,4H), 7.37(2CH,t,2H), 7.86(2CH,m,2H), 8.37(2CH,m,2H), 8.59(2CH,m,2H) ppm. MS:m/z:481.21 (M+1).

### Example 56 Effect of compounds on in vivo clotting time in animals

### Experimental Methods:

The control and model groups were gavaged with distilled water, and the positive clopidogrel group, and the test substance group were gavaged orally (i.g.) with drug solution in a volume of 0.1 mL/10 g for 4 consecutive days. 1 h after the last administration, all groups were injected with 0.15 mL/10 g of 0.3% ellagic acid solution via tail vein injection except for the blank group which was injected with physiological saline: 0.1 ml/10 g. After 5 min, glass capillaries were used for blood collection through the retro-orbital venous plexus, and the appearance time of coagulation filaments was recorded by glass capillaries at 10-second intervals. The experimental results were shown in Table 1.

Calculation equation for prolongation of clotting time: Prolongation of clotting time (%) = [(mean value of clotting time in blank or administered group/mean value of clotting time in model group)-1] x 100%

**Table 1 Effect of all test substances on clotting time in mice**

| Groups | Drug dose | Clotting time prolongation (%) |
|---|---|---|
| Normal control group | - | 38.40% |
| Model group | - | - |
| Clopidogrel | 100mg/kg | 36.80% |
| 1-1 | 100mg/kg | 43.30% |
| 1-2 | 100mg/kg | 49.90% |
| 1-4 | 100mg/kg | 39.70% |
| 1-5 | 100mg/kg | 41.90% |
| 1-6 | 100mg/kg | 42.40% |
| 1-7 | 100mg/kg | 41.70% |
| 1-8 | 100mg/kg | 44.90% |
| 1-11 | 100mg/kg | 39.30% |
| 1-12 | 100mg/kg | 43.30% |
| 1-13 | 100mg/kg | 39.80% |
| 1-15 | 100mg/kg | 42.00% |
| 1-16 | 100mg/kg | 38.00% |
| 1-17 | 100mg/kg | 44.10% |
| 1-18 | 100mg/kg | 42.90% |
| 1-19 | 100mg/kg | 60.40% |
| 1-22 | 100mg/kg | 40.70% |
| 1-23 | 100mg/kg | 38.10% |
| 1-24 | 100mg/kg | 36.70% |
| 1-26 | 100mg/kg | 40.40% |
| 1-27 | 100mg/kg | 39.50% |
| 1-30 | 100mg/kg | 39.80% |
| 1-32 | 100mg/kg | 48.40% |
| 1-34 | 100mg/kg | 53.50% |
| 1-37 | 100mg/kg | 32.64% |
| 1-38 | 100mg/kg | 37.51% |
| 1-39 | 100mg/kg | 37.23% |
| 1-42 | 100mg/kg | 41.40% |
| 1-45 | 100mg/kg | 43.10% |
| 1-48 | 100mg/kg | 38.56% |
| 1-49 | 100mg/kg | 38.72% |
| 1-54 | 100mg/kg | 37.20% |
| 1-55 | 100mg/kg | 38.60% |

As can be seen from Table 1, compared with the model group, the compounds of the present invention can prolong the clotting time in mice to different degrees. Compared with the positive drug clopidogrel group, the compounds of the present invention all showed strong anticoagulant effect.

### Example 57 Effect of compounds on in vivo bleeding time in animals

### Experimental Methods:

A normal control group (blank), positive control group (clopidogrel) and experimental groups were set up with 10 ICR mice (male, weighed about 25 g) in each group. Animals in each group were administered the corresponding drug solution by oral gavage (i.g.) at 0.1 ml/10g body weight, and the blank control group was given distilled water, once daily for 4 days. On the 4th day, 1h after the administration of the drug, the animals were anesthetized by intraperitoneal injection of sodium barbiturate at a dose of about 50 mg/kg, and 20 min later, the tails of the mice were cut at a distance of 3 mm from the tip of the tail, and at regular intervals, absorbent paper was used to dip the blood at the severed tails until no blood exuded, which was recorded as the bleeding time, and the results of the experiment were shown in Table 2.

Calculation equation for prolongation of bleeding time: Prolongation of bleeding time (%) = [Mean bleeding time in the administered group/mean bleeding time in the blank group- 1] × 100%

**Table 2 Effect of all test substances on bleeding time in mice**

| Groups | Drug dose | Bleeding time prolongation (%) |
|---|---|---|
| Normal control group | | 22.1% |
| Clopidogrel | 15mg/kg | > 579.89% |
| 1-1 | 100mg/kg | -8.89% |
| 1-2 | 100mg/kg | 6.50% |
| 1-4 | 100mg/kg | 16.40% |
| 1-5 | 100mg/kg | -13.60% |
| 1-6 | 100mg/kg | 4.80% |
| 1-7 | 100mg/kg | 15.10% |
| 1-8 | 100mg/kg | 12.20% |
| 1-11 | 100mg/kg | 20.60% |
| 1-12 | 100mg/kg | 27.30% |
| 1-13 | 100mg/kg | 15.60% |
| 1-15 | 100mg/kg | -4.70% |
| 1-16 | 100mg/kg | 11.00% |
| 1-17 | 100mg/kg | -6.20% |
| 1-18 | 100mg/kg | 13.30% |
| 1-19 | 100mg/kg | -38.00% |
| 1-22 | 100mg/kg | 19.83% |
| 1-23 | 100mg/kg | -19.20% |
| 1-24 | 100mg/kg | 20.60% |
| 1-26 | 100mg/kg | -13.30% |
| 1-27 | 100mg/kg | 23.50% |
| 1-30 | 100mg/kg | -9.70% |
| 1-32 | 100mg/kg | -9.00% |
| 1-34 | 100mg/kg | -2.10% |
| 1-37 | 100mg/kg | 48.80% |
| 1-38 | 100mg/kg | 28.40% |
| 1-39 | 100mg/kg | 16.90% |
| 1-42 | 100mg/kg | -13.40% |
| 1-45 | 100mg/kg | 18.60% |
| 1-48 | 100mg/kg | 26.40% |
| 1-49 | 100mg/kg | 15.30% |
| 1-54 | 100mg/kg | 13.00% |
| 1-55 | 100mg/kg | -3.10% |

As can be seen from Table 2, the positive control drug clopidogrel significantly prolonged the bleeding time in mice at 15 mg/kg, while the bleeding time of the compound of the present invention at 100 mg/kg was still much shorter than that of the clopidogrel group, and approximated that of the normal control group.

### Example 58 Effect of compound prophylactic administration on cerebral ischemia-reperfusion injury in rats

Experimental methods: SD rats were randomly divided into 6 groups, set as sham group, model control group, positive control drug ozagrel (6mg/kg) group, and test drug compound 1-1 high (9mg/kg), medium (3mg/kg), and low (1mg/kg) dose group. The administered groups were preadministered by tail vein injection, and the sham and model control groups were given an equal volume of saline, once a day for 3 consecutive days. Ten minutes after the last administration, the rat MCAO/R model was established, and reperfusion was performed 2 h after ischemia. 24 h after MCAO reperfusion, neurobehavioral scores were performed to determine the neurological function of rats; 2,3,5-triphenyltetrazolium chloride (TTC) staining method was used to determine the rate of cerebral infarction and cerebral water content in rats.

The results were as follows:
Table 3 Effect of prophylactic administration of compound 1-1 on the rate of cerebral infarction in rats with focal cerebral ischemia (MCAO)/reperfusion (data were expressed as

**Mean± SD, n=8)**

| Groups | Dose (mg/kg) | Cerebral infarction rate (%) |
|---|---|---|
| Sham | - | 0 |
| Model control group | - | 33.61±3.56 |
| Ozagrel group | 6mg/kg | 17.07±10.69** |
| Compound 1-1 high-dose group | 9mg/kg | 17.22±9.35** |
| Compound 1-1 medium-dose group | 3mg/kg | 22.74±9.12 |
| Compound 1-1 low-dose group | 1mg/kg | 25.20±9.71 |

| | | |
|---|---|---|
| ** P<0.01, compared with model control group | | |

As shown in Table 3, the rate of cerebral infarction in rats in the model group reached 33.61±3.56%, indicating successful modeling. Compared with the model group, the white infarction area on the surgical side of the rats in each administration group was reduced to different degrees, in which the positive drug ozagrel (6 mg/kg) group and the test drug compound 1-1 high-dose (9 mg/kg) group were able to significantly reduce the cerebral infarction rate of rats with focal cerebral ischemia/reperfusion injury (P<0.01).

**Table 4 Effect of prophylactic administration of compounds 1-1 on cerebral water content in rats with focal cerebral ischemia (MCAO)/reperfusion (data were expressed as Mean± SD, n=8)**

| Groups | Dose (mg/kg) | cerebral water content (%) |
|---|---|---|
| Sham | - | 79.82±0.42 |
| Model control group | - | 82.50±0.51## |
| Ozagrel group | 6mg/kg | 81.00±0.98** |
| Compound 1-1 high-dose group | 9mg/kg | 80.94±0.92** |
| Compound 1-1 medium-dose group | 3mg/kg | 81.57±0.87 |
| Compound 1-1 low-dose group | 1mg/kg | 81.81±0.78 |

| | | |
|---|---|---|
| ##P<0.01, compared with the sham group; **P<0.01, compared with the model control group | | |

As shown in Table 4, compared with the sham group, the cerebral water content in rats with focal cerebral ischemia-reperfusion in the model group was significantly higher than that in the sham group, indicating successful modeling; compared with the model group, the positive drug, ozagrel (6 mg/kg) group, and the test drug Compound 1-1 high-dose (9 mg/kg) group were able to significantly lower the cerebral water content in rats with focal cerebral ischemia-reperfusion injury (P < 0.01).

**Table 5 Effect of prophylactic administration of compound 1-1 on neurobehavior in rats with focal cerebral ischemia (MCAO)/reperfusion (data were expressed as Mean± SD, n=8)**

| Groups | Dose (mg/kg) | behavioral score |
|---|---|---|
| Model control group | - | 3.00±0.76 |
| Ozagrel group | 6mg/kg | 1.50±0.76** |
| Compound 1-1 high-dose group | 9mg/kg | 1.50±0.53** |
| Compound 1-1 medium-dose group | 3mg/kg | 2.00±0.76 |
| Compound 1-1 low-dose group | 1mg/kg | 2.63±0.74 |

| | | |
|---|---|---|
| ** P<0.01, compared with the model control group | | |

As shown in Table 5, after MCAO/R, rats in the model group had higher neurobehavioral scores, indicating that rats in the model group had severe neurological deficits. Compared with the model group, the positive drug ozagrel (6 mg/kg) group and the test drug compound 1-1 high-dose (9 mg/kg) group were able to significantly reduce the neurological function scores of rats with focal cerebral ischemia-reperfusion injury (P < 0.01).

Conclusion: Prophylactic administration of compound 1-1 reduces the rate of cerebral infarction and cerebral water content in rats with cerebral ischemia-reperfusion injury and improves neuromotor function in rats.

### Example 59 Inhibitory effect of compounds on myocardial ischemia-reperfusion injury caused by coronary artery ligation in rats

Experimental Methods: A model of myocardial ischemia/reperfusion (MI/R) injury caused by coronary artery ligation was established in rats, and animals were grouped into high-dose (20 mg/kg), medium-dose (10 mg/kg), low-dose (5 mg/kg) of the test drug, clopidogrel (7.5 mg/kg), sham, and model groups. Equal volume of 0.5% CMC-Na was administered at 0.5 ml/100 g body weight in both sham and model groups. The drug was administered by gavage (i.g.) once a day for 5 consecutive days. Modeling was performed 30 min after the last administration, and after modeling, 8 successfully modeled rats were selected from each group for subsequent experiments. The sham group was placed a wire under the left anterior descending branch of the coronary artery according to the model preparation method without ligation, and the other groups were modeled according to the model preparation method. The effects of the test drugs on the cardiac infarction rate and the biochemical indexes of creatine kinase (CK), lactate dehydrogenase (LDH), malondialdehyde (MDA), and superoxide dismutase (SOD) in plasma were detected, and TTC staining of the heart was performed to detect the effects of the test drugs on the area of myocardial infarction in MI/R rats.

The results were as follows:

**Table 6 Inhibitory effect of compound 1-1 on the rate of cardiac infarction in rats with myocardial ischemia-reperfusion injury (x̅±s, n=8)**

| groups | Dose (mg/kg) | Cardiac infarction rate (%) |
|---|---|---|
| Sham group model control group | - | 0 |
| | - | 22.51±6.96^{##} |
| | 20 | 7.2 7±1.91**^{△△} |
| Compound 1-1 group | 10 | 10.99±2.39** |
| | 5 | 12.12±4.45** |
| Clopidogrel group | 7.5 | 14.29±6.82* |

| | | |
|---|---|---|
| ##P<0.01 vs sham group; * P<0.05, ** P<0.01 vs model control group; ^{△}P<0.05,^{△△}P<0.01 vs clopidogrel group. | | |

**Table 7 Effect of compound 1-1 on SOD activity, MDA content, CK activity, LDH activity in plasma of rats with myocardial ischemia-reperfusion injury (x̅±s, n=8)**

| Groups | Dose (mg/kg) | SOD (U/mL) | MDA (nmol/mL) | CK (U/mL) | LDH (U/L) |
|---|---|---|---|---|---|
| Sham group | - | 61.28±6.34 | 4.35±0.45 | 0.14±0.076 | 741.63±401.95 |
| Model control group | - | 16.37±1.22^{##} | 31.10±2.15^{##} | 0.66±0.13^{##} | 15394.74±5644.70^{##} |
| Compound 1-1 group | 20 | 41.02±2.33** | 8.77±2.19**^{△△} | 0.08±0.047** | 4366.03±782.76**^{△△} |
| | 10 | 23.99±2.80** | 5.47±1.71**^{△△} | 0.27±0.11** | 3732.06±573.02**^{△△} |
| | 5 | 28.29±1.24** | 13.23±2.89** | 0.33±0.15** | 5406.70±458.93*^{△△} |
| Clopidogrel group | 7.5 | 40.92±1.67** | 15.90±1.37** | 0.32±0.20* | 8522.72±626.17* |

| | | | | | |
|---|---|---|---|---|---|
| ^{##}P<0.01 vs sham group;* P<0.05,** P<0.01 vs model control group; ^{△}P<0.05,^{△△}P<0.01 vs clopidogrel group. | | | | | |

**Table 8 Pathological scores of compound 1-1 on myocardial ischemia-reperfusion injury in rats (x̅±s, n=8)**

| Groups | Dose (mg/kg) | Pathological Score |
|---|---|---|
| Sham group | - | 1.50±0.76 |
| Model control group | - | 2.38±0.74^{#} |
| | 20 | 1.75±0.71 |
| Compound 1-1 group | 10 | 1.88±0.83 |
| | 5 | 2.00±0.53 |
| Clopidogrel group | 7.5 | 1.88±0.64 |

| | | |
|---|---|---|
| Note: ^{#}P<0.01 vs sham group. | | |

Conclusion: Compound 1-1 can affect the plasma biochemical indexes, improve the degree of myocardial lesions and inhibit the rate of myocardial infarction in rats with coronary artery ligation-induced myocardial ischemia/reperfusion injury, with the overall effect of the high-dose group of Compound 1-1 slightly better than that of the positive drug clopidogrel group.

### Example 60 Effect of a combination of compound and urokinase (i.v.) on ischemic brain injury induced by autologous thrombus and thrombin in rats

Experimental Methods: A model of cerebral ischemic injury in SD rats was prepared by embolizing the middle cerebral artery of rats with autologous thrombus and thrombin via the external carotid artery to the internal carotid artery and the rats were randomly divided into 5 groups according to behavioral scores, a sham group (only the external carotid artery was isolated); a model-control group (saline was given to both groups in equal volume); urokinase (5000 U/kg) group; compound 1-1 (22.5 mg) group; with 20 animals in each group (10 for detecting the rate of cerebral infarction, and the other 10 for pathological testing). Each group was administered slowly in the tail vein (1 ml per minute) 2h after modeling, and the decrease in blood flow was monitored after thrombin injection and 120 min after administration. Neurobehavioral scoring was performed on the rats after 24 h to determine the neurological function of the rats; 2,3,5-triphenyltetrazolium chloride (TTC) staining was used to determine the rate of cerebral infarction and cerebral water content, and hematoxylin-eosin (HE) staining was used to observe the pathological damage of the brain in rats administered with the drug 2 h after cerebral ischemia.

**Table 9 Effect of compound 1-1 (i.v) on cerebral blood flow after autologous thrombus and thrombin-induced cerebral ischemic injury in rats (Data were expressed as Mean± SD, n=10)**

| Groups | Dose | Percentage decrease after thrombin | Percentage decrease hours two after |
|---|---|---|---|
| | | injection (%) | administration (%) |
| Blank group | - | 0.04±4.01 | 0.33±0.33 |
| Model group | - | 44.40±5.22 | 41.18±5.67 |
| Compound 1-1 group | 9mg/kg | 39.18±4.75 | 29.24±2.67** |
| Urokinase group | 5000U/kg | 50.10±7.94 | 37.12±4.49## |
| Compound 1-1 + Urokinase group | 9mg/kg+5000U/kg | 34.39±4.94 | 21.65±3.76**## |

| | | | |
|---|---|---|---|
| ** P<0.01, compared with model control group; ## P<0.01, compared with own group after thrombin injection | | | |

**Table 10 Effect of Compound 1-1 (i.v) on neuroethology of rats (Data were expressed as Mean± SD, n=10)**

| Groups | Dose | Behavioral score |
|---|---|---|
| Model group | - | 3.00±0.82 |
| Compound 1-1 group | 9mg/kg | 1.70±0.82 |
| Urokinase group | 5000U/kg | 1.60±0.70* |
| Compound 1-1 + Urokinase group | 9mg/kg+5000U/kg | 1.40±0.52** |

| | | |
|---|---|---|
| * P<0.05; ** P<0.01, compared with model control group | | |

**Table 11 Effect of compound 1-1(i.v) on cerebral infarction rate and cerebral water content of autologous thrombus and thrombin-induced cerebral ischemic injury in rats mean±S.D., n=10)**

| Groups | Dose | Cerebral infarction rate | Cerebral water content (%) |
|---|---|---|---|
| Blank group | | - | 79.32±0.63 |
| Model group | | 29.94±6.18 | 82.20±1.33## |
| Compound 1-1 group | 9mg/kg | 14.99±10.91* | 80.32±0.97* |
| urokinase group | 5000U/kg | 9.72±9.89** | 79.78±0.72** |
| Compound 1-1 + Urokinase group | 9mg/kg+5000U/kg | 7.36±5.78** | 79.79±0.92** |

| | | | |
|---|---|---|---|
| ## P<0.01, vs. blank control group; * P<0.05, ** P<0.01, vs. model control group | | | |

**Table 12 Effect of compound 1-1(i.v) on pathological damage scores of cerebral ischemic areas in rats with autologous thrombus and thrombin-induced cerebral ischemic injury (mean±SD, n=10)**

| Groups | Dose | Pathology Score |
|---|---|---|
| Model group | - | 3.70±0.48 |
| Compound 1-1 group | 9mg/kg | 2.20±1.01 |
| Urokinase group | 5000U/kg | 1.75±0.86** |
| Compound 1-1 + Urokinase | 9mg/kg+5000U/kg | 1.60±1.15** |
| group | | |

| | | |
|---|---|---|
| * P<0.05, ** P<0.01, compared with model control group | | |

Results: Intravenous administration of Compound 1-1, urokinase and Compound 1-1+urokinase significantly reduced the percentage decrease in cerebral blood flow, the area of cerebral infarction and cerebral water content in rats with the model of cerebral ischemic injury caused by autologous thrombus and thrombin, and improved the pathological damage and behavioral changes, of which compound 1-1+ urokinase showed the most significant level of improvement in autologous thrombosis and thrombin-induced cerebral ischemic injury.

Conclusion: Compound 1-1(i.v) and its co-administration with urokinase can reduce the rate of cerebral infarction, cerebral water content and pathological damage in rats with ischemic brain damage caused by autologous thrombus and thrombin, improve the neuromotor function of rats, and show a better anti-ischemic brain damage effect.

### Example 61 Improvement of drug administration on focal permanent cerebral ischemic injury 3h post-ischemia

Experimental methods: The middle cerebral artery occlusion (MCAO) model in rats was prepared by the internal carotid artery wire embolization method. 48 Clean-grade male SD rats, weighed 250-300 g, were randomly divided into 6 groups according to their body weight and sex with 8 rats in each group, which were the compound 1-1 high-dose group, the compound 1-1 medium-dose group, the compound 1-1 low-dose group, the positive control group of 60 mg/kg of butylphthalide soft capsule, the sham group, and the ischemia-modeled control group (both of which were administered with an equal volume of 0.5% of CMC-Na). The administration of drug was started 3 h after the MCAO surgery and was given by gavage once a day in a volume of 0.5 ml/100 g body weight for 14 consecutive days. The animals were graded and scored for neurological deficits according to Bederson's method 1 h after the last administration, after which the rats were sacrificed by decapitation, the whole brain was removed and weighed, sections were stained with ttc, photographed, and the percentage of infarcts and the water content of the brain tissue were calculated.

Another 48 clean-grade male SD rats (100 rats were actually needed because the modeling rate was about 50%), weighed 250-300 g, were randomly divided into 6 groups each according to body weight with 8 rats in each group. Groups were administered and operated as before. The rats were sacrificed by decapitation 1h after the last drug administration, the whole brain was taken out and fixed in 10% formaldehyde solution, the brain coronal section was taken, dehydrated, paraffin-embedded, sectioned and stained with HE, and the histopathological changes were observed under the light microscope, and the degree of pathological damage was scored.

### Results:

**Table 13 15-day mortality statistics for rats**

| Groups | Dose (mg/kg) | Starting number of enrolled animal | Number of survivors after 15 days animal | Mortality (%) |
|---|---|---|---|---|
| Sham group | - | 8 | 8 | 0 |
| Model control group | - | 15 | 7 | 53.33 |
| Compound 1-1 group | 40 | 15 | 10 | 33.33 |
| | 20 | 15 | 11 | 26.67 |
| | 10 | 15 | 8 | 46.67 |
| Butylphthalid e group | 60 | 15 | 7 | 53.33 |

Results: Compound 1-1 middle and high dose groups significantly reduced the mortality of rats.

**Table 14 Mean body weight statistics of rats within 15 days**

| Groups | Dose (mg/kg) | Weight on day 1 (g) | Weight on day 5 (g) | Weight on day 15 (g) |
|---|---|---|---|---|
| Sham group | - | 286.88 | 316.13 | 360.25 |
| Model control group | - | 270.13 | 235.80 | 217.29 |
| | 40 | 274.93 | 288.25 | 316.80 |
| Compound 1-1 group | 20 | 281.93 | 253.67 | 281.10 |
| | 10 | 273.73 | 240.73 | 281.38 |
| Butylphthalide group | 60 | 281.00 | 234.27 | 276.75 |

Results: The body weight of rats in the sham group increased steadily in 15 days, and the body weight of rats in the model group showed an overall decreasing trend within 15 days. All of the administered groups had different degrees of effectiveness in maintaining the body weight of rats, as evidenced by the fact that the average body weight of the groups IS significantly higher than that of the model group after 15 days. Wherein, the compound 1-1 high-dose group showed significantly better body weight gain than the other dosing groups.

**Table 15 Statistics of neurological function scores in rats after 15 days**

| Groups | Dose (mg/kg) | Neurological function score on day 15 |
|---|---|---|
| Sham group | - | 0 |
| Model control group | - | 1.875 |
| | 40 | 0.5 |
| Compound 1-1 group | 20 | 0.75 |
| | 10 | 0.88 |
| Butylphthalide group | 60 | 1.25 |

Results: the administered group can significantly reduce the neurological function score of the rats after 15 days, which was manifested as slight hooking and limb asymmetry, and some of the rats were completely recovered with a neurological function score of 0. The neurological function score of the model group was significantly higher than that of the administered group, and some serious ones could reach 3 points, which was manifested as inability to walk in a straight line and constantly turning in circles.

Conclusion: Compound 1-1 has the effect of improving brain neurological function, in addition, it was found by TTC staining that the infarction rate and water content were significantly reduced in the compound 1-1 middle and high dose groups compared with the model group. Pathological examination showed that compared with the normal control group, the brain tissue of the model group showed obvious neuronal degeneration and necrosis, microglia proliferation, neuronal and interstitial edema, interstitial congestion, hemorrhage and other pathological changes; compared with the model group, the pathological changes in each group of the test product were reduced, of which the reduction of the high-dose group was the most obvious; the pathological changes of the positive control group were aggravated, especially the cortical neuronal degeneration and necrosis. This suggests that the test compound has a certain improvement effect on the pathological damage of permanent cerebral ischemia.

### Example 62 Compound Antibody Microarray Detection

Experimental methods: ICR male mice, weighed about 25g, were randomly divided into four groups: normal group, ellagic acid model group, 1-1 group, and 1-22 group, with four mice in each group. Mice in 1-1 group and 1-22 group were given 1-1, 100mg/kg and 1-22, 100mg/kg by gavage once a day respectively. 1 h after the third administration, except for the normal control group, the other three groups of mice were injected into the tail vein with 0.10 mL/10g of 0.3% ellagic acid saline solution, and 1 h after injection, whole blood was collected from each animal, and centrifuged (3,000 rpm/min) for 10 min after 30 min of standing, and then centrifuged to obtain the serum. The serum from each group of animal was mixed in an EP tube, transported at -80°C, and sent for antibody microarray detection.

### Results:

**Table 16**

| Proinflammatory factors-representative factor of acute inflammatory response | Normal group | Ellagic acid model group | 1-1 Group | 1-22 Group |
|---|---|---|---|---|
| IL-1b | 252.00 | 336.02^{#} | 312.69 | 268.76* |
| TNFa | 298.50 | 392.59^{#} | 386.79 | 290.22* |
| IL-6 | 1,236.00 | 28,515.60^{#} | 18,375.64* | 10,273.27* |
| RANTES/ccl5 | 2,109.00 | 3,505.11^{#} | 2,825.89* | 1,909.28* |

| | | | | |
|---|---|---|---|---|
| "#" indicates up-regulation of the expression of differential proteins compared with the normal group; "*" indicates down-regulation of the expression of differential proteins compared with the model group | | | | |

The results showed that the expression levels of IL-1b, TNFa, IL-6 and RANTES/ccl5 were significantly increased in the ellagic acid model group, suggesting that ellagic acid could cause acute inflammatory responses in the organism. Compound 1-1 can significantly down-regulate the expression levels of IL-6 and RANTES/ccl5, and compound 1-22 can significantly down-regulate the expression levels of IL-6 and RANTES/ccl5, suggesting that both 1-1 and 1-22 could decrease the acute inflammatory responses caused by ellagic acid, but compound 1-22 down-regulated more differential protein factors.

**Table 17**

| Interleukin factor | Normal group | Model group | 1-1 Group | 1-22 Group |
|---|---|---|---|---|
| IL-1α | 847.00 | 1,389.79^{#} | 1,118.99* | 837.49* |
| IL-1β | 252.00 | 336.02^{#} | 312.69 | 268.76* |
| IL-2 | 413.50 | 519.04^{#} | 565.56 | 464.49 |
| IL-3 | 820.00 | 1,243.37^{#} | 1,106.55 | 872.26* |
| IL-4 | 1,001.50 | 1,272.77^{#} | 1,367.20 | 961.11* |
| IL-5 | 240.50 | 350.44^{#} | 285.74* | 227.55* |
| IL-13 | 588.50 | 1,010.99^{#} | 800.30* | 620.73* |
| IL-6 | 1,236.00 | 28,515.60^{#} | 18,375.64* | 10,273.27* |

| | | | | |
|---|---|---|---|---|
| "#" indicates up-regulation of the expression of differential proteins compared with the normal group; "*" indicates down-regulation of the expression of differential proteins compared with the model group | | | | |

The results showed that all model groups could significantly up-regulate the expression of IL-1α, IL-1β, IL-2, IL-3, IL-4, IL-5, IL-6 and IL-13, compound 1-1 can down-regulate the expression levels of IL-1α, IL-5, IL-6 and IL-13, and compound 1-22 could down-regulate the levels of IL-1α, IL-1β, IL-3, IL-4, IL-5, IL-6 and IL-13. Compared with the model group, the number of down-regulated differential proteins in group 1-1 was four, and the number of down-regulated differential proteins in group 1-22 was seven. And the main down-regulated differential proteins interleukin factor in group I-1 and group 1-22 was Th2 cytokines.

**Table 18**

| Colony-stimulating factor | Normal group | Model group | 1-1 Group | 1-22 Group |
|---|---|---|---|---|
| Eotaxin /CCL11 | 3,443.50 | 5,143.45^{#} | 4,696.55 | 3,096.53* |
| MCP1/CCL2 | 1,374.00 | 2,811.28^{#} | 3,045.61 | 2,231.63* |
| MIP-3a/CCL20 | 1,293.00 | 2,004.86^{#} | 1,505.55* | 1,252.13* |
| RANTES/CCL5 | 2,109.00 | 3,505.11^{#} | 2,825.89* | 1,909.28* |

| | | | | |
|---|---|---|---|---|
| "#" indicates up-regulation of the expression of differential proteins compared with the normal group; "*" indicates down-regulation of the expression of differential proteins compared with the model group | | | | |

The results showed that all ellagic acid models significantly up-regulated the expression of these four inflammatory chemokines, compound 1-1 significantly down-regulated the expression of MIP-3a/CCL20 and RANTES/CCL5, and compound 1-22 significantly down-regulated the levels of all four inflammatory chemokines, indicating that both compounds 1-1 and 1-22 inhibited the inflammatory cell chemotaxis and inflammatory factor release.

**Table 19**

| Other factors | Normal group | Model group | 1-1 Group | 1-22 Group |
|---|---|---|---|---|
| IFNg | 221.00 | 360.42^{#} | 295.07* | 218.11* |
| CD40 | 299.50 | 661.58^{#} | 639.15 | 509.56* |

| | | | | |
|---|---|---|---|---|
| "#" indicates up-regulation of the expression of differential proteins compared with the normal group; "*" indicates down-regulation of the expression of differential proteins compared with the model group | | | | |

The results showed that the expression levels of IFNg and CD40 in the ellagic acid model group were significantly increased compared with those in the normal group, and that compound 1-1 significantly down-regulated IFNg, and compound 1-22 significantly down-regulated the expression of both IFNg and CD40, suggesting that 1-1 and 1-22 may inhibit antigen presentation and suppress immune cell activation.

Conclusion: Compared with the normal group, ellagic acid injection significantly increased the content of each tested cytokine, and the related biological functions involved included the positive regulation of leukocyte proliferation, differentiation and migration, the positive regulation of immune effects, the positive regulation of cell adhesion, and the positive regulation of acute inflammatory response, which led to acute inflammatory response of the organism. Compared with the model group, there was a significant down-regulation of differential proteins in both 1-1 and 1-22 groups, and both 1-1 and 1-22 obviously have the effects of anti-cytokine production, anti-acute inflammatory response and so on.

### Example 63 Preparation of Compound 1-1 Tablets

| Ingredients | Compound 1-1 100mg tablets |
|---|---|
| | mg/tablet |
| Compound 1-1 | 100 |
| Microcrystalline cellulose | 47 |
| Sodium cross-linked carboxymethyl cellulose | 6.4 |
| Hydroxypropyl Cellulose | 5.0 |
| Microcrystalline cellulose | 20 |
| Magnesium stearate | 3.0 |
| Silicon dioxide | 10.4 |
| Hydroxypropylmethylcellulose | 6.24 |
| Titanium oxide | 0.96 |
| Ferric oxide | 0.04 |
| Talc | 0.96 |
| Total weight | 200 |

According to the above prescription, compound 1-1, microcrystalline cellulose and crosslinked sodium carboxymethylcellulose were mixed and granulated with an aqueous solution of hydroxypropyl cellulose, and dried, microcrystalline cellulose and magnesium stearate were added to the granules after granulation and mixed, and the above granules were pressed to obtain plain tablets. The film coating solution made of titanium oxide, iron trioxide, talc and hydroxypropyl methyl cellulose aqueous solution was used in a coating machine to obtain film-coated tablets, with each tablet containing 100 mg of compound 1-1.

### Example 64 Preparation of Compound 1-1 Enteric Capsules

| Prescription | prescription dosage | role |
|---|---|---|
| Compound 1-1 | 400 g | Active ingredient |
| Microcrystalline cellulose | 212 g | filler |
| Lactose monohydrate | 212 g | filler |
| Hydroxypropyl Cellulose | 9.2 g | adhesive |
| Purified water | 174.8 g | Adhesive solvent |
| Magnesium stearate | 8.4 g | lubricant |

Compound 1-1, microcrystalline cellulose SH101, and lactose monohydrate were weighed according to the prescription amount for latter use; Compound 1-1, microcrystalline cellulose SH101, and lactose monohydrate were added into the pot of a wet mixing granulator, turning on the stirring, mixing for 5min, and then granulating: (1) binder preparation: appropriate amount of hydroxypropylmethylcellulose E5 was weighed and prepared to a 5% solution with water for latter use; (2) granulation: turning on the stirring, the binder solution was added by peristaltic pump, and the granulation time was 3min; (3) sieving and drying: the prepared wet particles were sieved by a swing granulator, and dried at 70°C in an electrothermal constant-temperature drying oven (turned over every 15min), with moisture ≤3%; the dried material was sieved by the swing granulator; the dosage of magnesium stearate was calculated according to the yield of the particles, and weighed and added into a laboratory hopper mixer together with the granulated material, stirred, and mixed for 5min; filled into enteric-coated capsule No.1, aluminum-plastic packaging.

### Example 65 Preparation of Compound 1-1 Lyophilized Powder Injection

| Prescription | Prescription dosage | Role |
|---|---|---|
| Compound 1-1 | 0.1-0.5g | Active ingredient |
| Mannitol | 0.03g-0.05g | excipient |
| Sodium hydroxide or | appropriate | pH adjuster |
| hydrochloric acid | amount | |
| Water for Injection | Add to 2 ml | Solvent |

The main ingredient compound 1-1 and mannitol were weighed according to the prescribed amount, added to 80% of the prescribed amount of water for injection, and stirred until dissolution; the pH value of the solution was measured, if the pH value is between pH6-7, the pH value is not adjusted, when exceeded, the pH value of the solution is adjusted with sodium hydroxide solution (or hydrochloric acid solution) to adjust the pH value of the drug solution, and then added with water for injection to the prescribed amount. The solution was first filtered through 0.45um microporous membrane, then filtered through 0.22um microporous membrane and filled. According to the set parameters of Lyophilization, the solution was lyophilized, pressed with plug, rolled with cap, and packed.

### Example 66 Preparation of Compound 1-24 for Injection

| | |
|---|---|
| Compound 1-24 | 1000 mg |
| Glycerin | 10 g |
| Sodium chloride | 3.5 g |
| Sodium citrate dihydrate | 150 mg |
| 0.1M hydrochloric acid or sodium hydroxide | appropriate amount |
| Water for injection is added to | 500ml |
| pH range of the solution | 5.0-6.5 |

To 80% of water for injection is added the prescribed amount of sodium chloride, sodium citrate dihydrate, glycerol and compounds 1-24, and stirred to dissolve.The pH value of the solution is adjusted to 5.0-6.5 with 0.1M hydrochloric acid or sodium hydroxide, and then the solution was filled with water for injection to constant volume. It was then dispensed into 10 ml glass vials and sterilized by high-pressure to obtain the injection solution of the compound. Each vial contains 20 mg of compound 1-24.

All documents referred to in the present invention are incorporated by reference herein as if each document is individually incorporated by reference. Further, it should be understood that upon reading the above teaching of the present invention, various modifications or alternations may be made to the present invention by those skilled in the art, and those equivalents also fall within the scope defined by the appended claims of the present application.

## Claims

1. A compound, wherein the compound is a compound of Formula I, or a pharmaceutically acceptable salt, a solvate, a hydrate, an isomer, or a prodrug thereof, 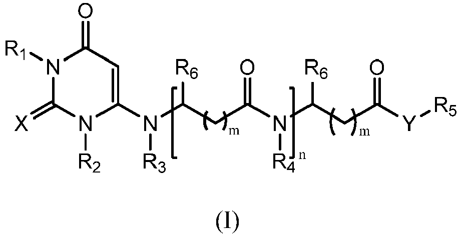 wherein,
R₁ and R₂ are each independently selected from the group consisting of: H, substituted or unsubstituted C1-C10 alkyl, C1-C10 haloalkyl, C3-C10 cycloalkyl, C3-C10 halocycloalkyl, C1-C10 alkoxy, C1-C10 haloalkoxy, -(C=O)-(C1-C10 alkyl), C2-C10 alkenyl, C2-C10 alkynyl, halogen, substituted or unsubstituted 5-10 membered heterocycloalkyl containing 1, 2 or 3 heteroatoms selected from N, O and S, substituted or unsubstituted C6-C10 aryl, substituted or unsubstituted 5-10 membered heteroaryl containing 1, 2 or 3 heteroatoms selected from N, O and S, -(C1-C6 alkylene)-(substituted or unsubstituted 5-10 membered heteroaryl containing 1, 2 or 3 heteroatoms selected from N, O and S ), -(C1-C6 alkylene)-(substituted or unsubstituted C6-C10 aryl);
X is selected from the group consisting of: O, S and NH;
Y is O;
R₃ is selected from the group consisting of: H and C1-C10 alkyl;
R₄ is selected from the group consisting of: H and C1-C10 alkyl;
R₅ is selected from the group consisting of: H, C1-C10 alkyl, substituted or unsubstituted C3-C10 cycloalkyl, substituted or unsubstituted C6-C10 aryl, substituted or unsubstituted 5-10 membered heterocycloalkyl containing 1, 2, or 3 heteroatoms selected from N, O, and S, and substituted or unsubstituted C5-C12 bridged cycloalkyl;
each R₆ is independently selected from the group consisting of: H, C1-C10 alkyl, -(C1-C6 alkylene)- (substituted or unsubstituted C6-C10 aryl);
each m is independently selected from the group consisting of: 0, 1, 2, 3, 4, 5 and 6;
n is selected from the group consisting of: 1, 2, 3 and 4;
the "substituted" each independently refers to being substituted by one or more substituents selected from the group consisting of: C1-C10 alkyl, -(C1-C6 alkylene)-piperazinyl, -(C1-C6 alkylene)- 5-10-membered heteroaryl containing 1, 2 or 3 heteroatoms selected from N, O and S, C1-C10 haloalkyl, C1-C10 alkoxy, C1-C10 alkylthiol, CN, nitro, -NRₐR_{b}, -(C=O)-NRₐR_{b}, halogen, hydroxy and oxo;
Rₐ and R_{b} are each independently selected from the group consisting of: H, C1-C10 alkyl, and C1-C10 haloalkyl;
optionally, the alkylene within the bracket is substituted with 1, 2 or 3 OH.

2. The compound according to claim 1, wherein,
R₁ and R₂ are each independently selected from the group consisting of: H, substituted or unsubstituted C1-C6 alkyl, -(C1-C6 alkylene)-(substituted or unsubstituted 5-10-membered heteroaryl containing 1, 2 or 3 heteroatoms selected from N, O and S), and -(C1-C6 alkylene)-(substituted or unsubstituted C6-C10 aryl);
X is selected from the group consisting of: O, S and NH;
Y is O;
R₃ is selected from the group consisting of: H and C1-C6 alkyl;
R₄ is H;
R₅ is selected from the group consisting of: H, C1-C6 alkyl, substituted or unsubstituted C3-C6 cycloalkyl, substituted or unsubstituted C6-C10 aryl, and substituted or unsubstituted C5-C12 bridged cycloalkyl;
each R₆ is independently selected from the group consisting of: H, C1-C6 alkyl, -(C1-C6 alkylene)- (substituted or unsubstituted C6-C10 aryl);
each m is independently selected from the group consisting of: 0, 1, 2, 3, 4, 5 and 6;
n is selected from the group consisting of: 1, 2, 3 and 4;
the "substituted" each independently refers to being substituted by one or more substituents selected from the group consisting of: C1-C6 alkyl, C1-C6 haloalkyl, CN and halogen.

3. The compound according to claim 1, wherein
R₁ and R₂ are each independently selected from the group consisting of: H, methyl, ethyl, propyl, isopropyl, butyl, isobutyl, tert-butyl, neopentyl, tert-pentyl, -CH₂-(1-to-6 R'-substituted or unsubstituted phenyl) and -CH₂-pyridyl;
each R' is independently selected from the group consisting of: methyl, ethyl, propyl, isopropyl, butyl, isobutyl, tert-butyl, neopentyl, tert-pentyl, F, Cl, Br, I and CN.

4. The compound according to claim 1, wherein, X is O or S.

5. The compound according to claim 1, wherein
R₃ is H;
R₄ is H;
R₅ is selected from the group consisting of: H, methyl, ethyl, propyl, isopropyl, butyl, tert-butyl, neopentyl, tert-pentyl, and the following groups: 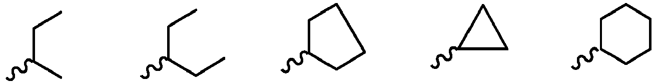 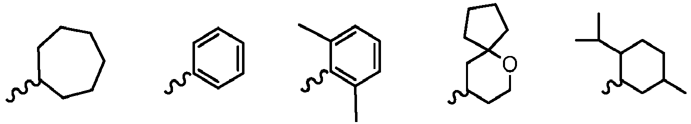 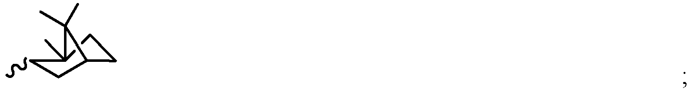
wherein 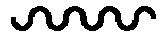 represents a linking bond to Y.

6. The compound according to claim 1, wherein the compound is selected from the group consisting of:
| No. | R₁ | R₂ | X | R₃ | R₆ | R₄ | Y | R₅ | m | n |
|---|---|---|---|---|---|---|---|---|---|---|
| 1-1 | CH₃ | CH₃ | O | H | H,H | H | O | CH₂CH₃ | 1,1 | 1 |
| 1-2 | CH₃ | CH₃ | O | H | H,H | H | O | CH(CH₃)₂ | 1,1 | 1 |
| 1-3 | CH₃ | CH₃ | O | H | H,H | H | O | H | 1,1 | 1 |
| 1-4 | CH₃ | CH₃ | O | H | H,H | H | O | 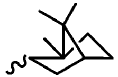 | 1,1 | 1 |
| 1-5 | CH₃ | CH₃ | O | H | H,H | H | O | 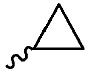 | 1,1 | 1 |
| 1-6 | CH₃ | CH₃ | O | H | H,H | H | O | 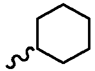 | 1,1 | 1 |
| 1-7 | CH₃ | CH₃ | O | H | H,H | H | O | 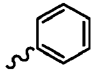 | 1,1 | 1 |
| 1-8 | CH₃ | CH₃ | O | H | H,H | H | O | 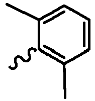 | 1,1 | 1 |
| 1-9 | CH₃ | CH₃ | S | H | H,H | H | O | CH₂CH₃ | 1,1 | 1 |
| 1-10 | CH₃ | CH₃ | S | H | H,H | H | O | H | 1,1 | 1 |
| 1-11 | CH₃ | CH₃ | S | H | H,H | H | O | 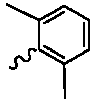 | 1,1 | 1 |
| 1-12 | CH₃ | CH₃ | O | H | H,H | H | O | 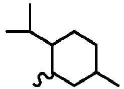 | 1,1 | 1 |
| 1-13 | CH₃ | 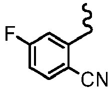 | O | H | H,H | H | O | CH(CH₃)₂ | 1,1 | 1 |
| 1-14 | CH₃ | 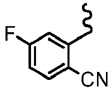 | O | H | H,H | H | O | H | 1,1 | 1 |
| 1-15 | CH₃ | 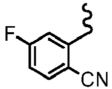 | O | H | H,H | H | O | 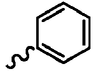 | 1,1 | 1 |
| 1-16 | CH₃ | 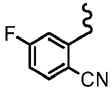 | O | H | H,H | H | O | 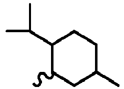 | 1,1 | 1 |
| 1-17 | CH₃ | 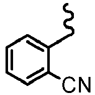 | O | H | H,H | H | O | CH₂CH₃ | 1,1 | 1 |
| 1-18 | CH₃ | 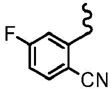 | O | H | H,H | H | O | CH₂CH₃ | 1,1 | 1 |
| 1-19 | CH₃ | 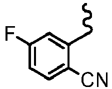 | O | H | H,H | H | O | 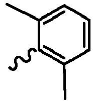 | 1,1 | 1 |
| 1-22 | CH₃ | CH₃ | O | H | 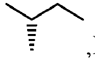 H | H | O | CH₂CH₃ | 0,1 | 1 |
| 1-23 | CH₃ | CH₃ | O | H | H,H | H | O | CH₂CH₃ | 4,4 | 1 |
| 1-24 | CH₃ | CH₃ | O | H | H,H | H | O | H | 4,4 | 1 |
| 1-26 | C₆H₅C H₂ | C₆H₅CH₂ | O | H | H,H | H | O | CH(CH₃)₂ | 1,1 | 1 |
| 1-27 | CH₃ | 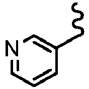 | O | H | H,H | H | O | CH(CH₃)₂ | 1,1 | 1 |
| 1-28 | CH₃ | C₆H₅CH₂ | O | H | H,H | H | O | CH₂CH₃ | 1,1 | 1 |
| 1-29 | CH₃ | C₆H₅CH₂ | O | H | H,H | H | O | H | 1,1 | 1 |
| 1-30 | CH₃ | C₆H₅CH₂ | O | H | H,H | H | O | 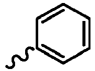 | 1,1 | 1 |
| 1-31 | CH₃ | 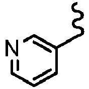 | O | H | H,H | H | O | H | 1,1 | 1 |
| 1-32 | CH₃ | 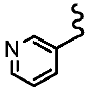 | O | H | H,H | H | O | 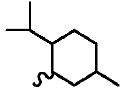 | 1,1 | 1 |
| 1-34 | 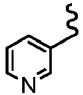 | 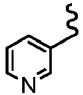 | O | H | H,H | H | O | CH(CH₃)₂ | 1,1 | 1 |
| 1-37 | CH₃ | CH₃ | O | H | 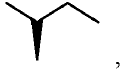 H | H | O | CH₂CH₃ | 0,1 | 1 |
| 1-38 | H | H | O | H | H,H | H | O | CH₂CH₃ | 4,4 | 1 |
| 1-39 | H | H | N H | H | H,H | H | O | CH₂CH₃ | 1,1 | 1 |
| 1-42 | CH₃ | CH₃ | O | H | 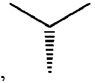 H | H | O | CH₂CH₃ | 1,0 | 1 |
| 1-45 | CH₃ | 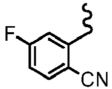 | O | CH₃ | H,H | H | O | CH(CH₃)₂ | 1,1 | 1 |
| 1-48 | CH₃ | CH₃ | O | H | H,H | H | O | CH₂CH₃ | 3,3 | 2 |
| 1-49 | CH₃ | CH₃ | O | H | 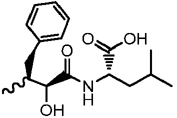 | | | | | |
| 1-54 | 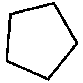 | 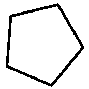 | O | H | H,H | H | O | CH₂CH₃ | 1,1 | 1 |
| 1-55 | 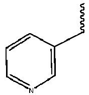 | 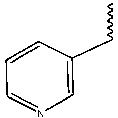 | O | H | H,H | H | O | CH₂CH₃ | 1,1 | 1 |

7. A preparation method of a compound according to claim 1, wherein the method comprises steps of: 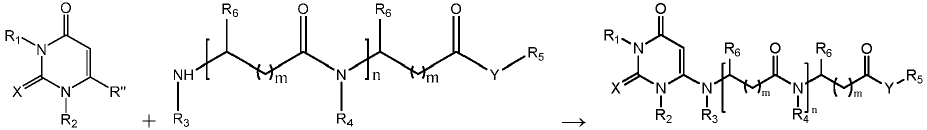 reacting 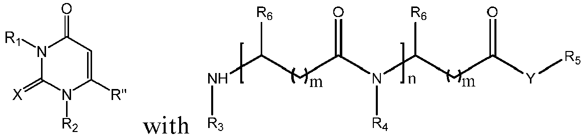 to obtain the compound of formula I;
wherein,
R" is halogen;
R₁, R₂, R₃, R₄, R₅, R₆, X, Y, m and n are as defined in claim 1.

8. A pharmaceutical composition, wherein the pharmaceutical composition comprises pharmaceutically acceptable carriers and a safe and effective amount of one or more compounds according to claim 1.

9. A use of the compound according to claim 1, wherein the use is selected from the group consisting of:
1) in the preparation of a medication for the treatment of infarction-related diseases;
2) in the preparation of a medication for thromboprophylaxis and/or thrombolytic therapy for thrombotic diseases, anti-edema therapy and/or immuno-anti-inflammatory therapy; and
3) in the preparation of a medication for the improvement of neuronal damage resulting from traumatic brain injury, cerebral hemorrhage, and/or brain tumor surgery.

10. The use according to claim 9, wherein the infarction-related diseases are selected from the group consisting of: neuronal damage of neural tissue resulting from acute ischemic stroke (cerebral infarction), myocardial infarction, and pulmonary embolism; and/or
the thrombotic diseases are selected from the group consisting of: cardiovascular diseases, cerebrovascular diseases, and peripheral vascular diseases.
